(19) Europäisches Patentamt
European Patent Office
Office européen des brevets

(11) EP 4 714 426 A1

(12) EUROPEAN PATENT APPLICATION
published in accordance with Art. 153(4) EPC

(43) Date of publication:
**25.03.2026 Bulletin 2026/13**

(21) Application number: **24807188.8**

(22) Date of filing: **13.05.2024**

(51) International Patent Classification (IPC):
***A61K 8/41*** *(2006.01)* ***A61K 8/36*** *(2006.01)*
***A61K 8/44*** *(2006.01)* ***A61Q 5/00*** *(2006.01)*

(52) Cooperative Patent Classification (CPC):
**A61K 8/36; A61K 8/41; A61K 8/44; A61Q 5/00**

(86) International application number:
**PCT/JP2024/017652**

(87) International publication number:
**WO 2024/237236 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.05.2023 JP 2023080877**

(71) Applicant: **Miyoshi Oil & Fat Co., Ltd.**
**Tokyo 124-0006 (JP)**

(72) Inventors:
- **YASHITA Akira**
  **Tokyo 124-0006 (JP)**
- **MIYAKE Shota**
  **Tokyo 124-0006 (JP)**
- **KAWAKAMI Hayato**
  **Iwakura-shi, Aichi 482-8511 (JP)**
- **KANEKO Kotaro**
  **Tokyo 124-0006 (JP)**
- **IBA Nobuyo**
  **Iwakura-shi, Aichi 482-8511 (JP)**
- **KAWAI Koji**
  **Tokyo 130-0012 (JP)**

(74) Representative: **Angerhausen, Christoph**
**Boehmert & Boehmert**
**Anwaltspartnerschaft mbB**
**Pettenkoferstrasse 22**
**80336 München (DE)**

# (54) ACTIVE INGREDIENT FOR HAIR

(57) Provided is an active ingredient for hair that efficiently permeates into hair, fixes therein and exhibits excellent repairment efficacy both on the surface and interior regions of the hair. Also provided is an active ingredient for hair that demonstrates excellent solubility of hair additives and exhibits excellent efficacies in terms of permeability and fixation of dyes and coloring agents, particularly for hair coloring treatment. The active ingredient for hair of the present invention contains components (A) and (B) of: (A) an amine or ammonium compound having a hydrogen-bonding functional group; and (B) a carboxylic acid or a salt thereof, wherein the ingredient contains the components (A) and (B) at a molar ratio from 0.01:1 to 10:1.

EP 4 714 426 A1

## Description

Technical Field

The present invention relates to an active ingredient for hair.

Background Art

The efficient permeation and fixation of various additives into, for example, tissues or materials, is crucial for enhancing the effects of the additives. Different types of active ingredients are widely utilized in various areas such as hair, skin, fiber, and wood.

In hair-related applications, hair additives used in, for example, treatments, conditioners, and coloring require enhanced permeation and fixation of the additives within the hair. However, conventional hair treatment agents (Patent documents 1 to 3) fail to provide sufficient permeation and fixation of additive components, and, for example, it is desired for such agents to have long-lasting functionality when they are used for hair repair or to have improved functionality of preventing discoloration after hair coloring when they are used for coloring.

The applicant proposed a cosmetic compounding agent, a skincare agent, and a hair treatment agent containing an amine compound or an amino acid (Patent documents 4 and 5). However, these prior documents do not specifically address enhancements in hair tactile sensation or the permeation and fixation thereof within the hair.

Prior Art Documents

Patent documents

Patent document 1: JP-A-2018-070461
Patent document 2: JP-A-2003-505405
Patent document 3: JP-A-2021-534103
Patent document 4: WO-A-2020/166678
Patent document 5: WO-A-2022/225048

Summary of Invention

Technical Problem

The present invention has been made in view of the above circumstances, and it is therefore an object of the present invention to provide an active ingredient for hair that efficiently permeates into hair, fixes therein, and exhibits excellent repairment efficacy both on the surface and interior regions of the hair. It is also an object of the present invention to provide an active ingredient for hair that demonstrates excellent solubility of hair additives and excellent permeability and fixation of hair additives, including excellent permeability and fixation of coloring agents used for hair additives for the purpose of hair coloring treatment.

Solution to Problem

The present invention provides solutions to the above-mentioned problems, as detailed below.

<1> An active ingredient for hair comprising components (A) and (B) of:

(A) an amine or ammonium compound having a hydrogen-bonding functional group; and
(B) a carboxylic acid or a salt thereof,

wherein the ingredient contains the components (A) and (B) at a molar ratio from 0.01:1 to 10:1.

<2> The active ingredient for hair according to <1>, wherein the component (A) is an amino acid represented by formula (1) or a salt thereof, the formula (1) being defined as
[Chemical formula 1]

$$R^1{}_lNH_mC(R^2)_2(R^3{}_nCOOX) \qquad (1)$$

wherein each $R^1$ represents a monovalent or bivalent hydrocarbon group having 1 to 22 carbon atoms, each $R^2$ independently represents a hydrogen atom, a monovalent or bivalent hydrocarbon group having 1 to 22 carbon atoms, a monovalent or bivalent nitrogen-containing group, a monovalent or bivalent oxygen-containing group, or a monovalent or bivalent sulfur-containing group, $R^3$ represents a bivalent hydrocarbon group having 1 to 22 carbon atoms, l is a integer of 0 to 2 and m is a integer of 0 to 2 provided that the sum of 1 and m is 2, and n is 0 or 1; wherein $R^1$ and $R^2$ together optionally form a ring having 3 to 22 carbon atoms; and wherein X represents a hydrogen atom or a monovalent cation.

<3> The active ingredient for hair according to <2>, wherein the amino acid is an amino acid (a) in which a ratio of the total number of primary or secondary amino groups to the number of carboxy groups (the total number of primary or secondary amino groups/ the number of carboxy groups) is greater than 1.

<4> The active ingredient for hair according to <1>, wherein the component (A) is an amine compound represented by formula (2) defined as
[Chemical formula 2]

$$N(H)_x(R^4)_y(R^5)_z \qquad (2)$$

wherein each $R^4$ independently represents a hydrocarbon group having at least one hydroxy group, each $R^5$ independently represents a hydrocarbon group having 1 to 22 carbon atoms, y is an integer of 1 to 3 and each of x and z is an integer of 0 to 2 provided that the sum of x, y, and z is 3.

<5> The active ingredient for hair according to any one of <1> to <4>, wherein the component (B) is a carboxylic acid or a salt thereof having a hydrogen-bonding functional group.

<6> The active ingredient for hair according to any one of <1> to <5>, wherein the components (A) and (B) forms a mixture that is liquid at 25°C.

<7> The active ingredient for hair according to any one of <1> to <6>, comprising an organic ammonium salt formed by the components (A) and (B).

<8> The active ingredient for hair according to <7>, wherein the organic ammonium salt is liquid at 25°C.

<9> The active ingredient for hair according to any one of <1> to <8>, wherein the ingredient is for use in a fixative agent for hair.

<10> A composition for hair comprising: the active ingredient for hair according to <1>; and a hair additive.

<11> The composition for hair according to <10>, wherein the hair additive is at least one additive selected from a dye, a coloring agent, a pigment, an astringent, and a reducing agent.

<12> The composition for hair according to <10> or <11>, comprising the hair additive and the active ingredient for hair at a compounding ratio of 10,000:1 to 1:10,000 by mass.

<13> A method for coloring hair and/or suppressing color deterioration of colored hair, comprising the steps of:

applying the composition for hair according to <11> or <12>, containing a dye as a hair additive, to hair; and leaving the hair with the applied composition for a predetermined duration.

<14> A method for coloring hair and/or suppressing color deterioration of colored hair, comprising:

a pretreatment step of applying a composition, containing the active ingredient for hair according to any one of <1> to <8>, to hair; and
a hair coloring step of applying a composition, containing a dye, to the hair after the pretreatment step, and then leaving the hair for a predetermined duration.

## Advantageous Effects of Invention

The active ingredient for hair according to the present invention efficiently permeates into hair, fixed therein, and demonstrates excellent repairment efficacy both on the surface and interior regions of the hair. Further, the active ingredient for hair according to the present invention demonstrates excellent solubility of hair additives, and is excellent in terms of permeability and fixation of hair additives, including excellent permeability and fixation of coloring agents used for hair additives for the purpose of hair coloring treatment.

## Brief Description of Drawings

FIG. 1 is a microscopic image illustrating the surface of damaged hair observed after undergoing bleaching.
FIG. 2 is a microscopic image illustrating the surface of damaged hair on which active ingredient 3 for hair is applied.
FIG. 3 is a microscopic cross-sectional image of hair, according to Working Example 17, after being colored and then washed once with shampoo.
FIG. 4 is a microscopic cross-sectional image of hair, according to Working Example 18, after being colored and then washed once with shampoo.
FIG. 5 is a microscopic cross-sectional image of hair, according to Working Example 24, after being colored and then washed once with shampoo.
FIG. 6 is a microscopic cross-sectional image of hair, according to Comparative Example 7, after being colored and then washed once with shampoo.
FIG.7 is a microscopic cross-sectional image of hair, according to Comparative Example 15, after being colored and then washed once with shampoo.

## Description of Embodiments

The embodiments of the present invention will be specifically described hereunder.
The number of carbon atoms as used herein represents an integer.

### 1. Active ingredient for hair

The active ingredient for hair of the present invention is an active ingredient for hair that permeates into hair, fixes therein, and exhibits excellent repairment efficacy on both the surface and interior regions of the hair. Examples of the hair include, but are not particularly limited to, human hairs (scalp hairs, mustaches/beards, eyebrows, and body hairs) and animal fibers and fur hairs (such as body fur hairs). Examples of the repair efficacy on both the surface and interior of hair include, but are not limited to, providing a good feel of use (such as finger-combing capability, moist feeling, and cohesiveness) by repairing hair surface cuticles, providing elasticity and resilience by repairing the hair from within, and providing the effects of suppressing waviness in the hair. Further, the ingredient may solubilize, permeate, and facilitate fixation of hair additives that are beneficial to the hair.
The active ingredient for hair of the present invention contains: component (A) that is an amine or ammonium compound having a hydrogen-bonding functional group; and component (B) that is a carboxylic acid or a salt thereof, wherein the ingredient contains the components (A) and (B) at a molar ratio from 0.01:1 to 10:1. In a preferable embodiment, the component (B) contains a hydrogen-bonding functional group.
The active ingredient, having a hydrogen-bonding functional group(s) not only in the component (A) but also in the component (B), exhibits superior affinity with organic or inorganic hair additives capable of bonding to or coordinating with hair or various hydrogen-bonding functional groups, thus enabling the active ingredient for hair to be permeated into the hair and fixed therein or allowing hair additive to permeate into the hair and fix it therein.
Examples of the hydrogen-bonding functional group contained in the components (A) and (B) include, but are not particularly limited to, an oxygen-containing group, a nitrogen-containing group, a sulfur-containing group, a phosphorus-containing group, and a hydrogen atom directly bonded to nitrogen.
It is preferred that the oxygen-containing group have less than or equal to 22 carbon atoms, more preferably less than or equal to 15 carbon atoms, and even more preferably less than or equal to 10 carbon atoms. Examples of the oxygen-containing group include, but are not particularly limited to, a hydroxy group-containing group, an alkoxy group-containing group, an acetoxy group-containing group, an acetyl group-containing group, an aldehyde group-containing group, a carboxy group-containing group, a carboxylate group-containing group, a urea group-containing group, a urethane group-containing group, an amide group-containing group, an imide group-containing group, an ether group-containing group, a carbonyl group-containing group, an ester group-containing group, an oxazole group-containing

group, a morpholin group-containing group, a carbamate group-containing group, a carbamic group-containing group, a carbamoyl group-containing group, a polyoxyethylene group-containing group, a tocopheryl group-containing group, a chroman group-containing group, a dihydropyran group-containing group, a glyceryl group-containing group, and a glyceryl ether group-containing group.

It is preferred that the nitrogen-containing group have less than or equal to 22 carbon atoms, more preferably less than or equal to 15 carbon atoms, and even more preferably less than or equal to 10 carbon atoms. Examples of the nitrogen-containing group include, but are not particularly limited to, a cyano group-containing group, a cyanato group-containing group, an isocyanate group-containing group, a nitro group-containing group, a nitroalkyl group-containing group, an amide group-containing group, a urea group-containing group, a urethane group-containing group, an imide group-containing group, a carbodiimide group-containing group, an azo group-containing group, a pyridyl group-containing group, an imidazole group-containing group, a pyrrolidyl group-containing group, a piperidyl group-containing group, a pyrrolyl group-containing group, a pyrazyl group-containing group, a triazole group-containing group, an isoquinolyl group-containing group, an oxazolyl group-containing group, a thiazolyl group-containing group, a morpholyl group-containing group, a guanidyl group-containing group, a pyrimidyl group-containing group, a piperazyl group-containing group, a triazyl group-containing group, a quinolyl group-containing group, an indole group-containing group, a quinoxalyl group-containing group, and an isoxazolyl group-containing group, a primary amino group-containing group, a secondary amino group-containing group, a tertiary amino group-containing group, a quaternary ammonium group-containing group, and an aminoalkyl group-containing group.

It is preferred that the sulfur-containing group have less than or equal to 22 carbon atoms, more preferably less than or equal to 15 carbon atoms, and even more preferably less than or equal to 10 carbon atoms. Examples of the sulfur-containing group include, but are not particularly limited to, a sulfate group-containing group, a sulfonyl group-containing group, a sulfonate group-containing group, a mercapto group-containing group, a thioether group-containing group, a thiocarbonyl group-containing group, a thiourea group-containing group, a thiocarboxy group-containing group, a thiocarboxylate group-containing group, a dithiocarboxy group-containing group, a dithiocarboxylate group-containing group, a sulfuric ester-containing group, a thiophene group-containing group, a thiazole group-containing group, a thiol group-containing group, a sulfo group-containing group, a sulfide group-containing group, a disulfide group-containing group, a thioester group-containing group, a thioamide group-containing group, a thiocarbamate group-containing group, and a dithiocarbamate group-containing group, and esters thereof.

It is preferred that the phosphorus-containing group have less than or equal to 22 carbon atoms, more preferably less than or equal to 15 carbon atoms, and even more preferably less than or equal to 10 carbon atoms. Examples of the phosphorus-containing group include, but are not particularly limited to, a phosphate group-containing group, a phosphorous acid group-containing group, a phosphonic acid group-containing group, a phosphinic acid group-containing group, a phosphonous acid group-containing group, a phosphinous acid group-containing group, a pyrophosphate group-containing group, a phosphate ester group-containing group, a phosphorous acid ester group-containing group, a phosphonic acid ester group-containing group, a pyrophosphate group-containing group, and esters thereof.

When any of the aforementioned oxygen-containing, nitrogen-containing, sulfur-containing, or phosphorus-containing groups includes a carbon atom, it is classified as a group having a hydrocarbon group, although this classification is not particularly limited. For instance, a hydroxy group-containing group refers to a group in which a hydroxy group is bonded to a hydrocarbon group.

(Component (A))

The component (A) is an amine or ammonium compound having a hydrogen-bonding functional group. Examples of the amine compound include, but are not particularly limited to, an amino acid, an amine (ammonia, a primary amine, a secondary amine, a tertiary amines), guanidine, and cyclic amines as typified by, for example, imidazole, pyridine, pyrrolidine, piperidine, pyrroline, pyrazine, triazine, isoquinoline, oxazoline, thiazoline, morpholine, pyrimidine, piperazine, triazine, quinoline, indoline, quinoxaline, and isooxazoline. Examples of the ammonium compound serving as the component (A) include salts of the above-listed amine compounds or quaternized amine compounds.

It is preferred that the component (A) be an amine compound, and among the above-listed amine compounds, amino acids and amines being more preferred, and amino acids being even more preferred.

[Hydrocarbon group]

Examples of the hydrocarbon group as used herein include, but are not limited to, a group having a structure established by subtracting at least one hydrogen atom from a hydrocarbon having 1 to 22 carbon atoms, and specific examples thereof include a saturated or unsaturated aliphatic hydrocarbon group, a saturated or unsaturated alicyclic hydrocarbon group, an aromatic hydrocarbon group and a hydrocarbon group of any combination of the preceding. The group may be monovalent or multivalent depending on the context of the application, and examples of the saturated or

unsaturated monovalent aliphatic hydrocarbon group include, but are not limited to, linear or branched alkyl, alkenyl, and alkynyl groups. Examples of the bivalent hydrocarbon group include groups having a structure established by subtracting two hydrogen atoms from a hydrocarbon.

It is preferred for enhancing the technical advantage of the present invention that at least one moiety capable of introducing a functional group in the component (A) is replaced with a hydrogen-bonding functional group-containing hydrocarbon group. Alternatively, it is preferred that a hydrogen atom(s) directly bonded to nitrogen constitutes a hydrogen-bonding functional group.

(Amino acid)

Preferred embodiments of the component (A) include an amino acid or a salt thereof. In the present invention, the amino acid encompasses a compound having a carboxy group (-COOH), as an acidic group, and an amino group (a primary amino group, a secondary amino group, or a tertiary amino group), as a basic group, in the molecule. Typical preferable examples thereof include, for example, a proteinogenic amino acid, a free amino acid in a living body, and an artificial amino acid. Preferred are a proteinogenic amino acid and a free amino acid in a living body.

An example of the amino acid salt includes an inner salt such as a carboxylate salt in which at least one of the carboxy groups of the amino acid is substituted by a cation (such as an alkali metal cation, an alkali earth metal cation, or an ammonium cation). When the amino acid has a plurality of nitrogen atoms, examples thereof include an amine salt in which the acid forms a salt with at least one nitrogen atom.

It is preferred that the amino acid or the salt thereof be represented by the following formula (1)
[Chem. 3]

$$R^1{}_lNH_mC(R^2)_2(R^3{}_nCOOX) \qquad (1)$$

wherein each $R^1$ represents a monovalent or bivalent hydrocarbon group having 1 to 22 carbon atoms, each $R^2$ independently represents a hydrogen atom, a monovalent or bivalent hydrocarbon group having 1 to 22 carbon atoms, a monovalent or bivalent nitrogen-containing group, a monovalent or bivalent oxygen-containing group, or a monovalent or bivalent sulfur-containing group, $R^3$ represents a bivalent hydrocarbon group having 1 to 22 carbon atoms, l is a integer of 0 to 2, m is an integer of 0 to 2 provided that the sum of l and m is 2, n is 0 or 2; wherein $R^1$ and $R^2$ together optionally form a ring having 3 to 22 carbon atoms; and wherein X represents a hydrogen atom or a monovalent cation.

In the formula (1), it is preferred that l be 0 or 1 and m be 1 or 2.

In the formula (1), $R^1$ is preferably an aliphatic hydrocarbon group.
In the formula (1), $R^2$ is preferably a hydrogen atom, a hydrocarbon group, or a nitrogen-containing, oxygen-containing, or sulfur-containing group having a hydrogen-bonding functional group(s).

It is preferred in terms of exhibiting affinity with water and hair and affinity with organic or inorganic materials and additives capable of bonding to or coordinating with hydrogen-bonding functional groups that the above-mentioned oxygen-containing, nitrogen-containing, or sulfur-containing group be contained therein as a hydrogen-bonding functional group. Of these functional groups, preferable examples of such oxygen-containing group include a hydroxy group-containing group, a carboxy group-containing group, a carboxylate group-containing group, and an amide group-containing group. Preferable examples of such nitrogen-containing group include an amide group-containing group, an imidazole group-containing group, a guanidyl group-containing group, an indole group-containing group, a primary amino group-containing group, a secondary amino group-containing group, and a tertiary amino group-containing group. Preferred examples of such sulfur-containing group include a thioether group-containing group, a thiol group-containing group, and a sulfide group-containing group.

Of these, more preferred is a guanidyl group-containing group.

In the formula (1), $R^3$ is preferably a saturated bivalent aliphatic hydrocarbon group (alkylene group).

Preferred embodiments of those represented by the formula (1) will be exemplified below.

In the formula (1), l is 0, m is 2, n is 0, and $R^2$ is a primary amino group or a nitrogen-containing group having at least two nitrogen atoms. Examples of such amino acids include, but are not particularly limited to, arginine, histidine, and lysine.

In the formula (1), $R^1$ and $R^2$ are each an oxygen-containing group where at least one of them contains a carboxy group. Examples of such amino acids include, but are not particularly limited to, glutamic acid and aspartic acid.

In the formula (1), at least one of $R^1$ and $R^2$ contains any one of a hydrocarbon group, an oxygen-containing group having a hydroxy group, a nitrogen-containing or sulfur-containing group having an amide group or a secondary amino group, or $R^1$ and $R^2$ together form a ring. Examples of such amino acids include, but are not particularly limited to, leucine, phenylalanine, proline, valine, tryptophan, serine, isoleucine, alanine, threonine, glutamine, asparagine, cysteine, and methionine.

The phrase "$R^1$ and $R^2$ may together form a ring having 3 to 22 carbon atoms" as used herein refers to that $R^1$ and

$R^2$ in combination form a ring of $R^1$, $R^2$, and C having nitrogen N in the ring in the unit of $R^1{}_lNH_mCR^2$ with the total number of carbons being 3 to 22, preferably 4 to 10.

In the formula (1), $R^1$ is a monovalent or bivalent saturated aliphatic hydrocarbon group which is preferably a saturated bivalent aliphatic hydrocarbon group. When it is a saturated bivalent aliphatic hydrocarbon group, it is preferred that $R^1$ and $R^2$ together form a ring.

It is preferred in terms of permeation into hair, fixation, and hair repairment efficacy that the component (A) be an amino acid wherein X in the (1) is a hydrogen atom.

The amino acid encompasses a compound having, in one molecule, at least one amino group (a primary, secondary, and/or tertiary amino group(s)) and at least one carboxy group (-COOH-) with the proviso that the amido group is not included in the amino group.

Preferred embodiments of the amino acids in terms of isoelectric point and the ratio between the number of amino groups and the number of carboxy groups include the following (hereafter referred to as amino acids (a) through (f)).

Amino acid (a):

A ratio of the total number of primary or secondary amino groups to the number of carboxy groups in the component (A) (Total number of primary or secondary amino groups/The number of carboxy groups) is greater than 1.

Examples of such amino acids include, but are not particularly limited to, arginine, histidine, lysine, and tryptophan.

Amino acid (b):

A ratio of the total number of primary or secondary amino groups to the number of carboxy groups in the component (A) (Total number of primary or secondary amino groups/The number of carboxy groups) is 1.

Examples of such amino acids include, but are not particularly limited to, leucine, isoleucine, phenylalanine, proline, valine, serine, alanine, threonine, glutamine, asparagine, aminobutyric acid, cysteine, glycine, and methionine.

Amino acid (c):

A ratio of the total number of primary or secondary amino groups to the number of carboxy groups in the component (A) (Total number of primary or secondary amino groups/The number of carboxy groups) is less than 1.

Examples of such amino acids include, but are not particularly limited to, glutamic acid and aspartic acid.

Amino acids are categorized according to their isoelectric points, and examples thereof include those above 7, between 4 and 7, and below 4.

Amino acid (d):

The component (A) has an isoelectric point above 7.

Examples of such amino acids include, but are not particularly limited to, those categorized as basic amino acids such as arginine (10.76), histidine (7.59), lysine (9.75), and aminobutyric acid (7.85). The numerals in the parentheses indicate the isoelectric points of the respective amino acids.

Examples of the amino acid having an isoelectric point above 7 include those where $R^2$ is represented by the following formula

[Chem. 4] $-R^{11}-R^{12}$

wherein $R^{11}$ represents a linear or branched saturated bivalent aliphatic hydrocarbon group having 1 to 10 carbon atoms and optionally containing a hydroxy group, and $R^{12}$ represents $-NH_2$, $-NHC(=NH)(NH_2)$, or an imidazolyl group.

Specific examples thereof include arginine, lysine, and histidine.

Amino acid (e):

The component (A) has an isoelectric point of 4 or more and 7 or less.

Examples of the amino acids having isoelectric points of 4 or more and 7 or less include, but are not particularly limited to, an amino acid having an alkyl-chain in $R^2$, an amino acid having a hydroxy group in $R^2$, an amino acid having sulfur in $R^2$, an amino acid having an amide group in $R^2$, an amino acid having an imino group in $R^2$, an amino acid having an aromatic group in $R^2$, and β-, γ-, δ-, and ε-amino acids.

The amino acid having an alkyl-chain in $R^2$ may be an amino acid, where $R^2$ is a hydrogen atom or a linear or

branched alkyl group having 1 to 10, preferably 1 to 4 carbon atoms. Specific examples thereof include, for example, glycine (5.97), alanine (6.00), valine (5.96), leucine (5.98), and isoleucine (6.02). The numerals in the parentheses indicate the isoelectric points of the respective amino acids.

The amino acid having a hydroxy group in $R^2$ may be an amino acid where $R^2$ is a linear or branched hydroxyalkyl group having 1 to 5, preferably 1 to 3, carbon atoms and having 1 to 3, preferably one, hydroxy group(s). Specific examples thereof include, for example, serine (5.68) and threonine (6.16). The numerals in the parentheses indicate the isoelectric points of the respective amino acids.

Examples of the amino acid having sulfur in $R^2$ include an amino acid where $R^2$ is represented by the following formula:

[Chem. 5] $(-R^{13}-)_{a1}(-S-)_{a2}R^{14}$

wherein $R^{13}$ represents a methylene group and $R^{14}$ represents a hydrogen atom or a methyl group, and a1 indicates any of 1 to 5, preferably of 1 to 3, and a2 indicates any of 1 to 4, preferably of 1 or 2. The a1 sets of $R^{13}$ and a2 sets of S may be arranged in any order.

Specific examples thereof include, for example, cysteine (5.07) and methionine (5.74). The numerals in the parentheses indicate the isoelectric points of the respective amino acids.

Examples of the amino acid having an amide group in $R^2$ include an amino acid where $R^2$ is represented by the following formula:

[Chem. 6] $-R^{15}-C(=O)NH_2$

wherein $R^{15}$ represents a linear or branched saturated bivalent aliphatic hydrocarbon group having 1 to 5, preferably 1 or 2 carbon atom(s).

Specific examples thereof include, for example, asparagine (5.41) and glutamine (5.65). The numerals in the parentheses indicate the isoelectric points of the respective amino acids.

Examples of the amino acid having an imino group in $R^2$ include a compound where N and $R^1$ together form a hetero ring. $R^1$ represents a saturated bivalent aliphatic hydrocarbon group having 3 to 4 carbon atoms and optionally having a hydroxy group, and forms a pyrrolidine ring or a piperidine ring. It is preferred that $R^1$ form a pyrrolidine ring.

Specific examples thereof include, for example, proline (6.30). The numerals in the parentheses indicate the isoelectric points of the respective amino acids.

Examples of the amino acid having an aromatic group in $R^2$ include an amino acid where $R^2$ is represented by the following formula:

[Chem. 7] $-R^{16}-R^{17}$

wherein $R^{16}$ represents a linear or branched saturated bivalent aliphatic hydrocarbon group having 1 to 5, preferably 1 or 2 carbon atoms and $R^{17}$ represents an aromatic hydrocarbon group having 6 to 10 carbon atoms and optionally having a substituted group, or a hetero ring group having 6 to 10 carbon atoms. $R^{17}$ is preferably a phenyl group, a hydroxyphenyl group, an indole group, or an imidazolyl group.

Specific examples thereof include, for example, phenylalanine (5.48), tyrosine (5.66), and tryptophan (5.89). The numerals in the parentheses indicate the isoelectric points of the respective amino acids.

Examples of the $\beta$, $\gamma$, $\delta$, or $\varepsilon$-amino acid include an amino acid where $R^3$ is a linear or branched saturated bivalent aliphatic hydrocarbon group having 1 to 4 carbon atoms.

Amino acid (f):

The component (A) has an isoelectric point of less than 4.

Examples of such amino acids include, but are not particularly limited to, those categorized as acidic amino acids such those in which $R^2$ is represented by the following formula

[Chem. 8] $-R^{18}COOH$

wherein $R^{18}$ represents a bivalent aliphatic hydrocarbon group having 1 to 10 carbon atoms.

Specific examples thereof include, for example, glutamic acid (3.22) and aspartic acid (2.77). The numerals in the parentheses indicate the isoelectric points of the respective amino acids.

Of the above amino acids (a) to (f), it is preferred in terms of permeation into hair, fixation, and hair repairment efficacy that the amino acid have a ratio of the total number of primary or secondary amino groups to the number of carboxy

groups of 1 or higher (Amino acid (a) and Amino acid (b)) and/or have an isoelectric point of 4 or more (amido acid (d) and amino acid (e)). Specific examples thereof include, for example, arginine, histidine, lysine, tryptophan, proline, aminobutyric acid, and serine.

It is also preferred from the above perspective that the amido acid have a ratio of the total number of primary or secondary amino groups to the number of carboxy groups of greater than 1 (amino acid (a)) or have an isoelectric point above 7 (amino acid (d)).

Specific examples thereof include arginine, histidine, lysine, and tryptophan. Of these, arginine, histidine, and lysine are preferred, and arginine and histidine are more preferred.

(Amine)

Preferable embodiments of the component (A) include an amine or a salt thereof. In the present invention, the salt of an amine encompasses salts of amines formed with organic or inorganic acids, as well as intramolecular salts comprising both an anion (such as a carboxylate, sulfonate, or phosphonate) and a cation within the one molecule.

It is preferred that the component (A) be an amine represented by the following formula (2)

[Chem. 9] $N(H)_x(R^4)_y(R^5)_z$ (2)

wherein each $R^4$ independently represents a hydrocarbon group having at least one hydroxy group, each $R^5$ independently represents a hydrocarbon group having 1 to 22 carbon atoms, the sum of x, y, and z is 3, y is an integer of 1 to 3, and each of x and z is an integer of 0 to 2.

Examples of hydrocarbon groups in $R^4$ of the formula (2) include those as defined above, among which preferred is a hydrocarbon group having 1 to 22 carbon atoms, and more preferred is a saturated or unsaturated aliphatic hydrocarbon group having 1 to 22 carbon atoms.

The above-mentioned saturated or unsaturated aliphatic hydrocarbon group, having 1 to 22 carbon atoms and at least one hydroxy group, has at least one hydroxy group and its hydrocarbon group may be linear or branched and preferably has 1 to 10 carbon atoms.

Examples of the linear or branched saturated or unsaturated aliphatic hydrocarbon group having at least one hydroxy group and preferably having 1 to 10 carbon atoms include: a monohydroxy saturated aliphatic hydrocarbon group having one hydroxy group (monohydroxy alkyl group); and a polyhydroxy saturated aliphatic hydrocarbon group having two or more hydroxy groups (polyhydroxy alkyl group). The hydrocarbon group may be linear or branched and preferably has 1 to 6, more preferably 1 to 4 carbon atoms, and it is preferred that the hydrocarbon group be a saturated hydrocarbon group.

Preferred examples of the linear or branched monohydroxy saturated aliphatic hydrocarbon group include, but are not particularly limited to, a monohydroxy alkyl group whose alkyl moiety has 1 to 10 carbon atoms, more preferably 1 to 6 carbon atoms, even more preferably 1 to 4 carbon atoms, and particularly preferably 1 to 3 carbon atoms.

Examples of the linear or branched polyhydroxy saturated aliphatic hydrocarbon group include, but are not particularly limited to, a di-, tri-, tetra-, penta-, hexa-, hepta- or octahydroxyalkyl group. The polyhydroxyalkyl group has preferably 2 to 8, more preferably 2 to 4, and even more preferably 2 to 3 hydroxy groups. The polyhydroxyalkyl group has an alkyl moiety having preferably 1 to 6, more preferably 1 to 4 carbon atoms.

Further, one preferable example is a branched polyhydroxyalkyl group represented by the following formula.

[Chem. 10]

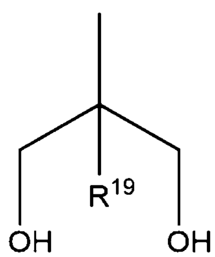

wherein $R^{19}$ represents a hydrogen atom, a linear alkyl group having 1 to 4 carbon atoms, or a linear monohydroxyalkyl group having 1 to 4 carbon atoms.

Among the aforementioned polyhydroxyalkyl groups, preferred groups include a 2,3-dihydroxypropane-1-yl group, a 1,3-dihydroxypropane-2-yl group, a 1,3-dihydroxy-2-methylpropane-2-yl group, a 1,3-dihydroxy-2-ethylpropane-2-yl group, a 1,3-dihydroxy-2-hydroxymethylpropane-2-yl group, and a pentahydroxyhexane-1-yl group. Among

these, a more preferred group is a 1,3-dihydroxy-2-hydroxymethylpropane-2-yl group.

Examples of the hydrocarbon group of $R^5$ in the formula (2) include those as mentioned above and it is preferred that the group be a saturated or unsaturated aliphatic hydrocarbon group having 1 to 12 carbon atoms, more preferably a saturated aliphatic hydrocarbon group having 1 to 8 carbon atoms, even more preferably a saturated aliphatic hydrocarbon group having 1 to 4 carbon atoms, and particularly preferably a saturated aliphatic hydrocarbon group having one carbon atom.

(Component (B))

The component (B) is a carboxylic acid or a salt thereof, and it is preferred that the component (B) be a carboxylic acid. The carboxylic acid is an organic acid having at least one carboxy group (-COOH) in the molecule. Examples of the carboxylic acid include, for example, a carboxylic acid having a hydrocarbon group that optionally has a hydrogen-bonding functional group. Examples of the carboxylic acid having a hydrocarbon group that optionally has a hydrogen-bonding functional group include, but are not particularly limited to, those having a carboxy group and a hydrocarbon group such as a saturated or unsaturated aliphatic hydrocarbon group, a saturated or unsaturated alicyclic hydrocarbon group, and an aromatic hydrocarbon group or the combination of the preceding, and the specific examples thereof include, for example, a saturated aliphatic carboxylic acid, an unsaturated aliphatic carboxylic acid, a saturated or unsaturated alicyclic carboxylic acid, an aromatic carboxylic acid, a saturated aliphatic hydroxycarboxylic acid, an unsaturated aliphatic hydroxycarboxylic acid, a saturated or unsaturated alicyclic hydroxycarboxylic acid, an aromatic hydroxycarboxylic acid, a carbonyl carboxylic acid, an alkyl ether carboxylic acid and a halogen carboxylic acid (Note that the number of carbon atoms in the carboxylic acids to be exemplified hereunder include the number of carbons in carboxy group(s)).

The saturated aliphatic carboxylic acid is comprised of a linear or branched saturated aliphatic hydrocarbon group and at least one carboxy group, and preferably has 1 to 22 carbon atoms. Examples of the saturated aliphatic carboxylic acid include, for example, a saturated aliphatic monocarboxylic acid having one carboxy group and a saturated aliphatic dicarboxylic acid having two carboxy groups. The saturated aliphatic monocarboxylic acid is comprised of a linear or branched saturated aliphatic hydrocarbon group and one carboxy group, and preferably has 1 to 22 carbon atoms. Particularly, preferred are a saturated aliphatic monocarboxylic acid selected from HCOOH and $CH_3(CH_2)_pCOOH$ (p is an integer of 0 to 16) and a saturated aliphatic monocarboxylic acid having a branched-chain. Specific examples thereof include, but are not particularly limited to, for example, formic acid, acetic acid, propionic acid, butyric acid, valeric acid, caproic acid, enanthic acid, caprylic acid, pelargonic acid, capric acid, lauric acid, myristic acid, pentadecylic acid, palmitic acid, margaric acid, stearic acid, arachidic acid, heneicosylic acid, behenic acid, isobutyric acid, 2-methylbutyric acid, isovaleric acid, 2-ethylhexanoic acid, isononanoic acid, isopalmitic acid and isostearic acid.

The saturated aliphatic dicarboxylic acid is comprised of a linear or branched saturated aliphatic hydrocarbon group and two carboxy groups, and preferably has 2 to 22 carbon atoms. Particularly, a saturated aliphatic dicarboxylic acid represented by $HOOC(CH_2)_qCOOH$ (q is an integer of 0 to 8) is preferred. Specific examples thereof include, but are not particularly limited to, for example, oxalic acid, malonic acid, succinic acid, glutaric acid, adipic acid, pimelic acid, suberic acid, azelaic acid, sebacic acid, and glutamic acid.

The unsaturated aliphatic carboxylic acid is comprised of a linear or branched unsaturated aliphatic hydrocarbon group and at least one carboxy group, and preferably has 3 to 22 carbon atoms. Examples of the unsaturated aliphatic carboxylic acid include, for example, an unsaturated aliphatic monocarboxylic acid having one carboxy group and an unsaturated aliphatic dicarboxylic acid having two carboxy groups.

The unsaturated aliphatic monocarboxylic acid is comprised of a linear or branched unsaturated aliphatic hydrocarbon group and one carboxy group, and preferably has 1 to 22 carbon atoms. Particularly, preferred is an unsaturated aliphatic monocarboxylic acid represented by $R^{21}CH=CH(CH_2)_rCOOH$ ($R^{21}$ represents a hydrogen atom or $CH_3(CH_2)_r$- (r is an integer of 0 to 7) and r represents an integer of 0 to 7). Specific examples thereof include, but are not particularly limited to, for example, acrylic acid, methacrylic acid, crotonic acid, palmitoleic acid, oleic acid, vaccenic acid, linoleic acid, linolenic acid, eleostearic acid, and arachidonic acid. The unsaturated aliphatic dicarboxylic acid is comprised of a linear or branched unsaturated aliphatic hydrocarbon group and two carboxy groups, and preferably has 1 to 4 carbon atoms. Specific examples thereof include, but are not particularly limited to, for example, maleic acid and fumaric acid.

The saturated or unsaturated alicyclic carboxylic acid is comprised of a non-aromatic saturated or unsaturated carbon ring and at least one carboxy group, and preferably has 6 to 20 carbon atoms. Particularly, a saturated alicyclic carboxylic acid having a cyclohexane ring skeleton is preferred. Examples of the saturated or unsaturated alicyclic carboxylic acid include a saturated or unsaturated alicyclic monocarboxylic acid having one carboxy group and a saturated or unsaturated alicyclic dicarboxylic acid having two carboxy groups.

Specific examples of the saturated or unsaturated alicyclic monocarboxylic acid include, but are not particularly limited to, cyclohexanecarboxylic acid. Specific examples of the saturated or unsaturated alicyclic dicarboxylic acid include, but are not particularly limited to, cyclohexanedicarboxylic acid.

The aromatic carboxylic acid is comprised of one or multiple aromatic rings and at least one carboxylic acid, and

preferably has 6 to 20 carbon atoms. Particularly, an aromatic carboxylic acid having a benzene ring skeleton is preferred. Examples of the aromatic carboxylic acid include an aromatic monocarboxylic acid having one carboxy group and an aromatic dicarboxylic acid having two carboxy groups.

Specific examples of the aromatic monocarboxylic acid include, but are not particularly limited to, benzoic acid and cinnamic acid. Examples of the aromatic dicarboxylic acid include, but are not particularly limited to, phthalic acid, isophthalic acid and terephthalic acid.

The saturated aliphatic hydroxycarboxylic acid is comprised of a linear or branched saturated aliphatic hydrocarbon group, at least one carboxy group, and at least one hydroxy group, and preferably has 2 to 24 carbon atoms. Particularly, a saturated aliphatic hydroxycarboxylic acid having 1 to 5 hydroxy groups and 2 to 7 carbon atoms is preferred. Examples of the saturated aliphatic hydroxycarboxylic acid include a saturated aliphatic hydroxy monocarboxylic acid having one carboxy group and a saturated aliphatic hydroxy di- or tricarboxylic acid having two or three carboxy groups.

The saturated aliphatic hydroxy monocarboxylic acid has preferably 2 to 20, more preferably 2 to 7 carbon atoms. It is preferred that the number of hydroxy groups be 1 to 5. Particularly, a saturated aliphatic hydroxy monocarboxylic acid represented by $(R^{22})_3C(C(R^{23})_2)_sCOOH$ is preferred (s represents an integer of 1 to 4, and the three $R^{22}$'s and the $2\times s$ $R^{23}$'s each independently represents a hydrogen atom or a hydroxy group. The total number of hydroxy groups is 1 to 5). Specific examples thereof include, but are not particularly limited to, for example, glycolic acid, lactic acid, glyceric acid, hydroxyacetic acid, hydroxybutyric acid, 2-hydroxydecanoic acid, 3-hydroxydecanoic acid, 12-hydroxystearic acid, dihydroxystearic acid, cerebronic acid, leucine acid, mevalonic acid, pantoic acid, gluconic acid, galactonic acid, mannonic acid, arabinonic acid, fructuronic acid, tagathuronic acid, and aldonic acid.

It is preferred that the saturated aliphatic hydroxy di- or tricarboxylic acid has 4 to 22 carbon atoms. It is preferred that the number of hydroxy groups be 1 to 3. Particularly, a saturated aliphatic hydroxy di- or tricarboxylic acid represented by $HOOCC(R^{24}R^{25})C(R^{26}R^{27})C(R^{28}R^{29})COOH$ is preferred ($R^{24}$ to $R^{29}$ each independently represents a hydrogen atom, a hydroxy group or a carboxy group, the total number of hydroxy groups is 1 to 2, and the total number of carboxy groups is 1 to 2.) Specific examples thereof include, but are not particularly limited to, for example, tartronic acid, malic acid, tartaric acid, citramalic acid, citric acid and isocitric acid

The unsaturated aliphatic hydroxycarboxylic acid is comprised of a linear or branched unsaturated aliphatic hydrocarbon group, at least one carboxy group, and at least one hydroxy group, and preferably has 3 to 22 carbon atoms. Specific examples thereof include, but are not particularly limited to, ricinolic acid, ricinoleic acid, and ricineraidic acid.

The saturated or unsaturated alicyclic hydroxycarboxylic acid is comprised of a non-aromatic saturated or unsaturated carbon ring, at least one carboxy group, and at least one hydroxy group, and preferably has 4 to 20 carbon atoms. Particularly, an alicyclic hydroxycarboxylic acid having a six-membered ring skeleton and 1 to 4 hydroxy groups is preferred. Specific examples thereof include, but are not particularly limited to, for example, hydroxycyclohexanecarboxylic acid, dihydroxycyclohexanecarboxylic acid, quinic acid (1,3,4,5-tetrahydroxycyclohexanecarboxylic acid), shikimic acid, glucuronic acid, galacturonic acid, mannuronic acid, iduronic acid, and guluronic acid. Further, hydroxy group-containing cyclic lactones may also be preferably used; specific examples thereof include, but are not particularly limited to, for example, ascorbic acid and erythorbic acid.

The aforementioned aromatic hydroxycarboxylic acid is comprised of one or multiple aromatic rings, at least one carboxy group, and at least one hydroxy group, and preferably has 6 to 20 carbon atoms. Particularly, an aromatic hydroxycarboxylic acid having a benzene ring skeleton and 1 to 3 hydroxy groups is preferred; Specific examples thereof include, but are not particularly limited to, for example, salicylic acid, hydroxybenzoic acid, dihydroxybenzoic acid, trihydroxybenzoic acid (gallic acid), hydroxymethylbenzoic acid, vanillic acid, syringic acid, protocatechuic acid, gentisic acid, orsellinic acid, mandelic acid, benzylic acid, atrolactic acid, phloretic acid, coumaric acid, umbellic acid, caffeic acid, ferulic acid and sinapic acid.

The carbonyl carboxylic acid is a carboxylic acid having a carbonyl group(s) in a molecule and having 3 to 22 carbon atoms; preferred is a carbonyl carboxylic acid having 1 to 2 carbonyl groups and 3 to 7 carbon atoms. Particularly, preferred is a carbonyl carboxylic acid represented by $CH_3((CH_2)_tCO(CH_2)_u)COOH$ (t and u each represent an integer of 0 to 2). Specific examples thereof include, but are not particularly limited to, for example, pyruvic acid.

The alkyl ether carboxylic acid is a carboxylic acid having an ether group(s) in the molecule, having 2 to 22 carbon atoms, and having a polyoxyalkylene alkyl ether carboxylic acid; preferred is an alkyl carboxylic acid having 1 to 2 ether groups and 2 to 12 carbon atoms. Particularly, preferred is an alkoxy carboxylic acid or polyoxyethylene alkyl ether carboxylic acid represented by $CH_3(CH_2)_vO(CH_2)_wCOOH$ (v and w each represents an integer of 0 to 4), and more preferred is an alkoxy carboxylic acid. Specific examples thereof include, but are not particularly limited to, for example, methoxyacetic acid, ethoxyacetic acid, methoxybutyric acid, and ethoxybutyric acid.

The halogen carboxylic acid is preferably a halogen carboxylic acid having 2 to 22 carbon atoms.

Specific examples thereof include, but are not particularly limited to, for example, halogen-substituted halogen carboxylic acids such as trifluoroacetic acid, trichloroacetic acid, tribromoacetic acid, pentafluoropropionic acid, pentachloropropionic acid, pentabromopropionic acid, perfluorononanoic acid, perchlorononanoic acid, and perbromononanoic acid.

Of the above-exemplified carboxylic acids, in view of exhibiting efficacies in terms of permeation into hair, fixation within the hair structure, repairment of both the surface and interior regions of the hair, and improved tactile sensation of the hair due to its affinity for hair and hair additives, it is preferred that the component (B) have a hydrogen-bonding functional group, and it is more preferred that the component (B) be a carboxylic acid containing an oxygen-containing group, of which preferred examples are a hydroxy group, a carboxy group, and a carboxylate group.

In respect of hair repairment efficacy and improving tactile sensation by virtue of the active ingredient, preferred are a saturated aliphatic monocarboxylic acid, a saturated aliphatic dicarboxylic acid, an unsaturated aliphatic dicarboxylic acid, a saturated aliphatic hydroxy monocarboxylic acid, a saturated aliphatic hydroxy di- or tricarboxylic acid, and a lactone with a hydroxy group; and more preferred are a saturated aliphatic monocarboxylic acid and a saturated aliphatic hydroxy di- or tricarboxylic acid.

In respect of permeability and fixation of the active ingredient for hair and the hair additive in the composition for hair, preferred are a saturated aliphatic monocarboxylic acid, a saturated aliphatic dicarboxylic acid, an unsaturated aliphatic dicarboxylic acid, a saturated aliphatic hydroxy monocarboxylic acid, a saturated aliphatic hydroxy di- or tricarboxylic acid, an aromatic hydroxycarboxylic acid, and a lactone with a hydroxy group; and more preferred are a saturated aliphatic hydroxy monocarboxylic acid and a saturated aliphatic hydroxy di- or tricarboxylic acid; and even more preferred a saturated aliphatic hydroxy di- or tricarboxylic acid.

In respect of permeability and fixation of the hair additive in the composition for hair which employs an oxidation dye, preferred are a saturated aliphatic monocarboxylic acid, a saturated aliphatic dicarboxylic acid, an unsaturated aliphatic dicarboxylic acid, a saturated aliphatic hydroxy monocarboxylic acid, a saturated aliphatic hydroxy di- or tricarboxylic acid, and a lactone with a hydroxy group; more preferred are a saturated aliphatic hydroxy monocarboxylic acid and a saturated aliphatic hydroxy di- or tricarboxylic acid; and even more preferred is a saturated aliphatic hydroxy di- or tricarboxylic acid.

In respect of exhibiting efficacies in terms of permeation into hair, fixation, and repairment of both the surface and interior regions of the hair, it is preferred that the above carboxylic acids have 1 to 12, more preferably 1 to 8, carbon atoms.

The active ingredient for hair of the present invention contains the components (A) and (B), and the term also encompasses those having an organic ammonium salt formed from the components (A) and (B). The organic ammonium salt is formed by a cation derived from the component (A) and an anion derived from the component (B).

The surface of the hair or the like is negatively charged, and therefore the cationic organic ammonium salt is likely to be adsorbed on the surface of the hair, which therefore makes it suitable in respect of fixation of the active ingredient for hair and the hair additive and hair repairment efficacy.

It is preferred in terms of exhibiting efficacies in terms of permeation into hair, fixation, and repairment of both the surface and interior regions of the hair that the active ingredient for hair according to the present invention be one of the combinations of the components (A) and (B) as specified below.

[A] The component (A) is an amino acid while the component (B) is at least one of a saturated aliphatic monocarboxylic acid, a saturated aliphatic dicarboxylic acid, an unsaturated aliphatic dicarboxylic acid, a saturated aliphatic hydroxy monocarboxylic acid, a saturated aliphatic hydroxy di- or tricarboxylic acid, an aromatic hydroxycarboxylic acid, and a cyclic lactone with a hydroxy group.

[B] The component (A) is an amino acid of the amino acids (a) and (d) as listed above, while the component (B) is a saturated aliphatic hydroxy monocarboxylic acid or a saturated aliphatic hydroxy di- or tricarboxylic acid.

[C] The[B] is further limited such that the component (A) is at least one selected from arginine, lysine, and histidine, while the component (B) is a saturated aliphatic hydroxy monocarboxylic acid or a saturated aliphatic hydroxy di- or tricarboxylic acid.

[D] The[B] is further limited such that the component (A) is at least one selected from arginine, lysine, and histidine, while the component (B) is at least one selected from lactic acid, gluconic acid, malic acid, tartaric acid, and citric acid.

[E] The component (A) is an amino acid of the amino acids (a) and (e) as listed above, while the component (B) is a saturated aliphatic hydroxy monocarboxylic acid or a saturated aliphatic hydroxy di- or tricarboxylic acid.

[F] The[E] is further limited such that the component (A) is tryptophan, while the component (B) is a saturated aliphatic hydroxy monocarboxylic acid or a saturated aliphatic hydroxy di- or tricarboxylic acid.

[G] The[E] is further limited such that the component (A) is tryptophan, while the component (B) is at least one selected from lactic acid, gluconic acid, malic acid, tartaric acid, and citric acid.

[H] The component (A) is an amino acid of the amino acids (b) and (e) as listed above, while the component (B) is a saturated aliphatic hydroxy monocarboxylic acid or a saturated aliphatic hydroxy di- or tricarboxylic acid.

[I] The[H] is further limited such that the component (A) is at least one selected from leucine, isoleucine, phenylalanine, proline, valine, serine, alanine, threonine, glutamine, asparagine, cysteine, glycine, and methionine, while the component (B) is a saturated aliphatic hydroxy monocarboxylic acid or a saturated aliphatic hydroxy di- or tricarboxylic acid.

[J] The[H] is further limited such that the component (A) is at least one selected from leucine, isoleucine, phenylalanine, proline, valine, serine, alanine, threonine, glutamine, asparagine, cysteine, glycine, and methionine, while the component (B) is at least one selected from lactic acid, gluconic acid, malic acid, tartaric acid and citric acid.
[K] The component (A) is an amino acid of the amino acids (b) and (d) as listed above, while the component (B) is a hydroxycarboxylic acid.
[L] The[K] is further limited such that the component (A) is aminobutyric acid, while the component (B) is a saturated aliphatic hydroxy monocarboxylic acid or a saturated aliphatic hydroxy di- or tricarboxylic acid.
[M] The[K] is further limited such that the component (A) is aminobutyric acid, while the component (B) is at least one selected from lactic acid, gluconic acid, malic acid, tartaric acid, and citric acid.
[N] The component (A) is an amino acid of the amino acids (c) and (f) as listed above, while the component (B) is a saturated aliphatic hydroxy monocarboxylic acid or a saturated aliphatic hydroxy di- or tricarboxylic acid.
[O] The[N] is further limited such that the component (A) is glutamic acid or aspartic acid, while the component (B) is a saturated aliphatic hydroxy monocarboxylic acid or a saturated aliphatic hydroxy di- or tricarboxylic acid.
[P] The[N] is further limited such that the component (A) is glutamic acid or aspartic acid, while the component (B) is at least one selected from lactic acid, gluconic acid, malic acid, tartaric acid, and citric acid.

The active ingredient for hair of the present invention may have the components (A) and (B) at any compounding molar ratio which is not particularly limited but in respect of exhibiting efficacies in terms of permeation into hair, fixation, and repairment of both the surface and interior regions of the hair, the molar ratio therebetween may be 0.01:1 to 10:1, preferably 0.1:1 to 2:1, more preferably 0.5:1 to 2:1, and even more preferably 0.5:1 to 1.5:1. In respect of exhibiting excellent fixation property of a mixture of the components (A) and (B) or an organic ammonium salt formed by the components (A) and (B), preferable examples of the combinations and molar ratios of the components (A) and (B) are as specified below:

1) Molar ratio of components (A) and (B) is 0.5:1, where the component (A) is arginine and the component (B) is acetic acid
2) Molar ratio of the components (A) and (B) is 0.5:1, where the component (A) is arginine and the component (B) is lactic acid
3) Molar ratio of components (A) and (B) is 0.5:1, where the component (A) is arginine and the component (B) is gluconic acid.
4) Molar ratio of the components (A) and (B) is 1:1, where the component (A) is arginine and the component (B) is adipic acid
5) Molar ratio of the components (A) and (B) is 1:1, where the component (A) is arginine and the component (B) is succinic acid
6) Molar ratio of the components (A) and (B) is 1:1, where the component (A) is arginine and the component (B) is fumaric acid.
7) Molar ratio of the components (A) and (B) is 1:1, where the component (A) is arginine and the component (B) is malic acid.
8) Molar ratio of the components (A) and (B) is 1:1, where the component (A) is arginine and the component (B) is tartaric acid.
9) Molar ratio of the components (A) and (B) is 1:1, where the component (A) is arginine and the component (B) is citric acid.
10) Molar ratio of the components (A) and (B) is 1.5:1, where the component (A) is arginine and the component (B) is citric acid.
11) Molar ratio of the components (A) and (B) is 0.5:1, where the component (A) is arginine and the component (B) is gallic acid.
12) Molar ratio of the components (A) and (B) is 0.5:1, where the component (A) is arginine and the component (B) is ascorbic acid.
13) Molar ratio of the components (A) and (B) is 1:1, where the component (A) is 2-amino-2-hydroxymethyl-1,3-propanediol (Tromethamine) and the component (B) is lactic acid.
14) Molar ratio of the components (A) and (B) is 0.5:1, where the component (A) is histidine and the component (B) is lactic acid.
15) Molar ratio of the components (A) and (B) is 1.5:1, where the component (A) is histidine and the component (B) is citric acid.
16) Molar ratio of the components (A) and (B) is 1:1, where the component (A) is proline and the component (B) is lactic acid.
17) Molar ratio of the components (A) and (B) is 1:1, where the component (A) is aminobutyric acid and the component (B) is lactic acid.
18) Molar ratio of the components (A) and (B) is 1:1, where the component (A) is serine and the component (B) is lactic

acid.

The active ingredient for hair of the present invention is excellent in exhibiting efficacies in terms of permeation into hair, fixation, and repairment of both the surface and interior regions of the hair owing to its hydrogen-bonding functional groups in, for example, amino groups and carboxy groups in the component(s) (A) and/or (B). Preferable combinations of the component (A) and the component (B) are determined such that the total number of amino groups in the components (A) and (B) is equal to or higher than the total number of carboxy and carboxylate groups in the components (A) and (B), and it is preferred that the components (A) and (B) be fully neutralized or that un-neutralized amino acids remain therein when the components (A) and (B) form organic salts. That is, it is preferred that the ratio of the total number of amino groups in the components (A) and (B) to the total number of carboxy and carboxylate groups in the components (A) and (B) (Total number of amino groups/Total number of carboxy and carboxylate groups) be 1 or higher.

The active ingredient for hair of the present invention may take any state which is not particularly limited, but examples of which include, for example, a solid, a liquid, a gel, and a cream. However, it is preferred that the ingredient be liquid at 25°C in respect of exhibiting efficacies in terms of permeation into hair, fixation, and repairment of both the surface and interior regions of the hair, and allowing uniform application or adhesion to the surface of a subject for use. When the active ingredient for hair of the present invention is liquid at 25°C, such non-volatile and liquid active ingredient for hair and its dilution will not cause any usage problems such as a problem that the crystals thereof may be precipitated, aggregated, or solidified. When the active ingredient for hair is used for hair, the effects of the active ingredient for hair of the present invention and the effects of an additive(s) dissolved therein can be exhibited immediately after the use of the agent in a more effective manner and for a long period of time, due to the fact that even after other solvents mixed therein and water have volatilized, the ingredient can still be evenly applied to and coat the surface of the hair as a liquid. Further, the active ingredient for hair that is liquid at 25°C may serve as a solvent for a hair additive and exhibit the advantage of promoting permeation and fixation of the hair additive. As shown above, regarding the active ingredient for hair of the present invention, it is preferred that the components (A) and (B) form a mixture that is liquid at 25°C, and that an organic ammonium salt formed by the components (A) and (B) be liquid at 25°C.

Further, in terms of safety and damage control of hair or the like, it is preferred that the active ingredient for hair of the present invention have a pH level of 4 to 9, more preferably 5 to 9. Although not particularly limited, for example, when the pH level thereof is 4 to 9, the compounding molar ratio between the component (A) and the component (B) is 0.1:1 to 2:1.

The active ingredient for hair of the present invention has excellent affinity with various hair additives capable of hydrogen bonding, and is particularly excellent in affinity with proteins containing hard keratin with a high cysteine content. Therefore, for example, the one that is excellent in exhibiting efficacies in terms of permeation into hair, fixation, and hair repairment with a hydrogen-bonding functional group is suitable.

The active ingredient for hair of the present invention itself exhibits excellent effects in terms of permeation into keratin, fixation, and hair repairment. Particularly, when the component (A) and/or the component (B) in the active ingredient are/is a useful component(s) (for example, arginine) for hair, the active ingredient for hair containing both the component (A) and the component (B) exhibits more superior effects in terms of permeation, fixation, and hair repairment and more enhanced effects of the useful component(s) compared to the one containing only the component (A) or the component (B) alone.

(Subject for use)

The active ingredient for hair in the present invention may be applied to any suitable subject, without particular limitation, and examples thereof include human hair, animal fibers, and body hair. In particular, the active ingredient demonstrates enhanced suitability for materials containing keratin, and preferred applications encompass human hair (such as scalp hair, mustaches, beards, eyebrows, and body hair), animal fibers, and fur hair (including body fur hairs).

Among these, the active ingredient may be suitably used for materials containing hard keratin, such as hair. For example, when it is applied to hair, the active ingredient exhibits excellent efficacy in permeating it not only into the surface layer but also into the interior of the hair, being fixed therein, and repairing both the surface and interior regions of the hair. For example, in respect of cuticle repair efficacy and the tactile properties of the hair surface, the active ingredient may provide enhanced finger-combing capability, cohesiveness, a moisturized feel, and smoothness, while in respect of hair interior repairment, it is effective in providing elasticity/resilience, delivering a voluminous impression, waviness correction, and providing a glossy impression. Furthermore, it is useful in terms of the physiological activity of hair when the component (A) is an amino acid, particularly when it is a proteinogenic amino acid or a free amino acid in the body, and especially when it is a proteinogenic amino acid.

(Hair additive)

The active ingredient for hair in the present invention may be combined for use with any hair additive that is neither component (A) nor component (B), and is suitable for application to hair. The hair additive is a useful component for hair, and examples thereof include, but are not particularly limited to, a dye, a coloring agent, a pigment, an astringent, a reducing agent, an organic compound such as a resin that is a raw material for adhesive, carbon black, an inorganic compound such as a metal oxide, a protein and derivatives thereof (such as an amino acid and derivatives thereof, a protein hydrolysate, a peptide), a carbohydrate and its derivatives (such as sugars and their derivatives, sugar fatty acid esters), a lipid and derivatives thereof (such as ceramides, phospholipids and their derivatives, and sterols and their derivatives), alcohols (such as an alcohol, a polyhydric alcohol, and a fatty acid ester of alcohol), water, a solvent, an anionic surfactant, a nonionic surfactant, a cationic surfactant, an amphoteric surfactant, a cationic polymer, a water-soluble polymer, a viscosity regulator, a gloss imparting agent, a higher alcohol, a multivalent alcohol, a higher fatty acid, amidoamines, a hydrocarbon, a wax, esters, a silicone derivative, a physiologically active ingredient, extracts, an antioxidant, a sequestrant, a preservative, an ultraviolet absorber (organic and inorganic), a perfume, a moisturizer, carbons, minerals, salts, a neutralizer, a pH adjuster, resin particles, a natural plant extract ingredient, a seaweed extract ingredient, a crude drug ingredient, a refreshing agent, an insect repellent, and an enzyme.
Of these, it is preferred that the active ingredient be combined for use with a dye, a coloring agent, or a pigment, more preferably with a dye, of which the more suitable one is a combination with an oxidation dye.

(Composition for hair containing active ingredient for hair)

Examples of the composition for hair containing the active ingredient for hair of the present invention include a composition for hair that contains an active ingredient for hair and a component designed to be permeated thereinto and fixed within the hair. Examples of such component designed to be permeated thereinto and fixed within the hair include, but are not particularly limited to, those listed above (as hair additives).
The hair additive may be solubilized to enhance the efficacies of the hair additive in terms of permeation, fixation, and hair repairment to thereby enhance the effects of the hair additive.
The composition for hair containing the active ingredient of the present invention requires only the components (A) and (B) contained therein. When the composition for hair is produced, it may be produced either by first preparing the active ingredient containing the components (A) and (B) and subsequently mixing it with the remaining components to create the composition for hair, or by separately mixing the components (A) and (B) with the remaining components to create the composition for hair.

(Molecular size)

The composition for hair containing the active ingredient of the present invention is designed to permeate the active ingredient for hair deep into the interior of a target for use to fix the active ingredient for hair. In view of this respect, although the active ingredient for hair may have any molecular size, which is not particularly limited, it is preferred that the total sum of the components (A) and (B) have a molecular weight of 2,000 or lower. It is particularly preferred that the molecular weight be 1,000 or less in respect of exhibiting efficacies in terms of permeation and fixation thereof into hair that contains hard keratin having a rich content of cysteine.

(Compounding ratio)

The compounding ratio between the active ingredient for hair and the hair additive is not particularly limited and may range from, for example, 10,000:1 to 1:10,000 based on mass ratio. Preferably, the range is from 100:1 to 1:100, more preferably from 10:1 to 1:10, even more preferably from 5:1 to 1:5, and particularly preferably from 5:1 to 1:1, based on mass ratio. The amount of the active ingredient for hair as used herein is a value calculated in terms of the mass of the components (A) and (B).
It is also preferred in terms of permeation and fixation of the hair additive into hair that the compounding ratio between the active ingredient for hair and the hair additive be determined such that the active ingredient for hair is excessive. An excessive amount of the active ingredient for hair solubilizes the hair additive and facilitates bonding and coordination of the hydrogen-bonding functional groups in the active ingredient with both the hair and the hair additive, thereby efficiently fixing the hair additive to the hair.
When the active ingredient for hair is intended to be applied to a subject for use in a form of a composition for hair containing the ingredient, the composition may contain the active ingredient for hair at any amount which is not particularly limited, but the content may be, for example, from 0.01% to 50% by mass. It is preferred that the content be 0.1% by mass or higher, more preferably 0.5% by mass or higher, and even more preferably 1% by mass or higher. It is also preferred that

the content be 20% by mass or lower, and more preferably 10% by mass or lower. The amount of the active ingredient for hair as used herein is a value calculated in terms of the mass of the components (A) and (B).

(Fixative agent for hair)

The present invention provides a fixative agent for hair which is intended for use in, for example, human hairs such as hair, beard, eyebrows, eyelashes, nose hair, ear hair, armpit hair, and body hair although they are not particularly limited.

The fixative agent for hair of the present invention utilizes the component (A) and the component (B), and it is preferred in terms of safety that either one or both of them employ(s) a compound as listed in Japanese standards of quasi-drug ingredients (JSQI), Japanese Pharmaceutical Excipients, Japanese standards of quasi-drug additives, Japanese pharmacopoeia (JP), Japanese pharmaceutical codex (JPC), Japanese pharmaceutical excipients (JPE), or Japan's specifications and standards for food additives (JSFA). Examples of such component (A) include, but are not particularly limited to, amino acids such as glycine, alanine, arginine, aspartic acid, histidine, cysteine, proline, serine, tryptophan, tyrosine, methionine, aminobutyric acid, aminohexanoic acid, cystine, glutamic acid, isoleucine, phenylalanine, threonine, tryptophan, methionine, valine, and theanine; and monoethanolamine, diethanolamine, triethanolamine, 2-amino-2-hydroxymethyl-1,3-propanediol, 2-amino-2-methyl-1-propanol, 2-amino-2-methyl-1,3-propanediol, and choline. Of these, preferred are amino acids and 2-amino-2-hydroxymethyl-1,3-propanediol.

Examples of such component (B) include, but are not particularly limited to, malic acid, tartaric acid, acetic acid, caprylic acid, capric acid, lauric acid, myristic acid, palmitic acid, stearic acid, oleic acid, linoleic acid, isostearic acid, hydroxystearic acid, lactic acid, glycolic acid, succinic acid, citric acid, fumaric acid, ascorbic acid, benzoic acid, nicotinic acid, and gallic acid. Of these, it is preferable to employ a natural compound in respect of safety.

The active ingredient for hair of the present invention is useful as a fixative agent for hair, and is suitable for fixing the hair additive. It is particularly useful for coloring hair. The ingredient containing the components (A) and (B) allows a dye to be efficiently fixed therein and exhibits excellent effects in coloring hair, even the interior of the hair, while it also exhibits excellent effects in suppressing color deterioration after coloring the hair.

For this reason, the active ingredient for hair of the present invention and the composition for hair may be applied to, for example, human hair, animal fibers, and body hair, and examples thereof include, but are not particularly limited to, a hair treatment agent, cosmetics, a hair dyeing agent (a dye such as an oxidative dye, an acid dye, a neutral dye, a non-ionic dye, a basic dye), a paint, and a surface treatment agent. Examples of the hair treatment agent include, but are not particularly limited to, a hair shampoo, a scalp shampoo, a conditioner-integrated shampoo, a conditioning shampoo, a color shampoo, a hair soap, an anti-fading shampoo, a dry shampoo (non-rinse shampoo), a hair conditioner, a coloring conditioner, a hair cleansing product, a treatment product, a color treatment product, a non-rinse treatment product, an out bath treatment product, a conditioner, an out bath product, a styling agent, a set lotion agent, a hair manicure, a hair oil, a hair spray, a hair mist, a mousse, a foam, a hair gel, a hair cream, a hair wax, a hair liquid, a hair tonic, a hair growing agent, a hair nourishing agent, a treatment product for scalp, a mascara, an eyelash cosmetic, a product for eyebrow, and an eyebrow pencil. Examples of the hair dyeing agent include, but are not particularly limited to, a hair coloring agent, a hair manicure, a hair dye, a gray hair dye, a fashion dye, an acid color, a color rinse, a color treatment, a color shampoo, a hair mascara, a hair foundation, a hair color spray, and a color stick.

Among the aforementioned applications, the use of arginine, proline, or serine as the component (A) is preferred for fixing a dye in hair.

Further, when the active ingredient for hair of the present invention is used as a composition for hair, a hair coloring composition may be used to color hair or suppress discoloration of colored hair, and an exemplary method for this process may involve a step of applying the above composition, containing a dye as a hair additive, to hair, and a step of leaving the hair with the applied composition for a predetermined duration. The composition may be applied to the hair using any method, which is not particularly limited, but examples thereof include immersion.

The active ingredient for hair of the present invention may also be used as a pretreatment agent for coloring hair, and in such a case, the active ingredient for hair of the present invention may color hair or suppress discoloration of colored hair. An exemplary method for this process may involve a pretreatment step of applying the composition, containing the active ingredient for hair, to the hair and a hair coloring step of applying a composition, containing a dye, to the hair after the pretreatment step, and then leaving the hair for a predetermined duration. The composition, containing the active ingredient for hair, and the composition, containing a dye, may be applied to the hair using any method, which is not particularly limited, but examples thereof include immersion.

## Examples

The present invention will be described in more detail below with reference to examples, but the present invention is not limited to these examples.

(Active ingredient for hair)

Active ingredients 1 to 22 for hair

The compounds as listed below were used for the active ingredients for hair shown in Tables 1 to 4 and were formulated at molar ratios shown in the Tables. Visually observed states (liquid or solid) of the respective active ingredient for hair at 25°C were as shown in Tables 1 to 4.

Arginine produced by Wako Pure Chemical Corporation
Tromethamine (2-amino-2-hydroxymethyl-1,3-propanediol) produced by Tokyo Chemical Industry Co., Ltd.
Histidine produced by FUJIFILM Wako Pure Chemical Corporation
Proline produced by FUJIFILM Wako Pure Chemical Corporation
γ-aminobutyric acid produced by FUJIFILM Wako Pure Chemical Corporation
Serine produced by FUJIFILM Wako Pure Chemical Corporation
Acetic acid produced by FUJIFILM Wako Pure Chemical Corporation
Lactic acid produced by FUJIFILM Wako Pure Chemical Corporation
Gluconic acid produced by FUJIFILM Wako Pure Chemical Corporation
Adipic acid produced by FUJIFILM Wako Pure Chemical Corporation
Succinic acid produced by FUJIFILM Wako Pure Chemical Corporation
Fumaric acid produced by FUJIFILM Wako Pure Chemical Corporation
Malic acid produced by FUJIFILM Wako Pure Chemical Corporation
Tartaric acid produced by FUJIFILM Wako Pure Chemical Corporation
Citric acid produced by FUJIFILM Wako Pure Chemical Corporation
Gallic acid produced by FUJIFILM Wako Pure Chemical Corporation
Ascorbic acid produced by FUJIFILM Wako Pure Chemical Corporation
Arginine hydrochloride produced by FUJIFILM Wako Pure Chemical Corporation
Diethyl sebacate produced by FUJIFILM Wako Pure Chemical Corporation
Benzyl alcohol produced by FUJIFILM Wako Pure Chemical Corporation
Tetrabutylammonium bromide produced by Lion Akzo Co., ltd. (Arquad 44-100)
1-butyl-3-methylimidazolium hydroxide prepared by mixing equal molar amounts of 1-butyl-3-methylimidazolium chloride (produced by Tokyo Chemical Industry Co., Ltd) and potassium hydroxide

1. Evaluation on hair repairment efficacy (cuticle repairment efficacy) and tactile sensation improvement by virtue of the active ingredient

1.1 Hair repairment efficacy (cuticle repairment efficacy) and tactile sensation improvement by sensory evaluation

A sample containing the components (A) and (B) (an aqueous solution in which the total sum of the components (A) and (B) was 80% by mass) was prepared and used as a sample.

Black hairs were subjected to the bleach treatment to prepare damaged hairs. As an operation of the bleach treatment, 1g of bleaching agent, prepared at the formulation shown below, was applied to 1g by mass of untreated black hair (10 cm, manufactured by Beaulax Co., Ltd), which was allowed to stand still for 30 minutes and then sufficiently rinsed with ion-exchange water at 40°C. This bleach treatment operation was repeated 5 times to prepare damaged hairs.

<Bleaching agent>

| | |
|---|---|
| 30% Hydrogen Peroxide: | 9.6 parts by mass |
| 28% Ammonia Water: | 4.0 parts by mass |
| Monoethanolamine: | 1.5 parts by mass |
| Ammonium bicarbonate: | 1.0 parts by mass |
| Ion-exchange water: | Remainder |
| Total: | 100 parts by mass |

One minute was spent in evenly applying 10.0 g of each sample to a bundle of the damaged hairs weighing about

10 g using a brush, and then the bundle of the hairs was left for 5 minutes. After leaving it for 5 minutes, the bundle of the damaged hairs was rinsed with ion-exchange water of 40°C for 30 seconds, and the hair bundle that had been rinsed was then dried with a towel and further subjected to air drying for a day. The air-dried hair was evaluated for the aspects of: elasticity/resilience, non-waviness, finger-combing capability, moist feeling, and cohesiveness from the perspective of superiority or inferiority to a test hair bundle(s) uncoated with the sample. The same operation was performed on samples of an 80% by mass aqueous solution containing only the component (A), an 80% by mass aqueous solution containing only the component (B), and water as comparative examples. Ten panel members were randomly selected regardless of age and sex for evaluation. The following four grades were used for evaluation.

◎: Superior
◦: Favorable
Δ: No change
×: Poor

[Table 1A]

| | Active ingredient for hair | Component (A) | | | | | | |
|---|---|---|---|---|---|---|---|---|
| | | Compound | $R^1$ | $R^2$ | $R^3$ | l | m | n |
| Working Example 1 | Active ingredient for hair 1 | L-Arginine | - | H, $(CH_2)_3NHC(NH)NH_2$ | - | 0 | 2 | 0 |
| Working Example 2 | Active ingredient for hair 2 | | | | | | | |
| Working Example 3 | Active ingredient for hair 3 | | | | | | | |
| Working Example 4 | Active ingredient for hair 4 | | | | | | | |
| Working Example 5 | Active ingredient for hair 5 | | | | | | | |
| Working Example 6 | Active ingredient for hair 6 | | | | | | | |
| Working Example 7 | Active ingredient for hair 7 | Tromethamine | - | - | - | - | - | - |
| Working Example 40 | Active ingredient for hair 23 | L-Histidine | - | H, $CH_2$(1H-imidazol-4-yl) | - | 0 | 2 | 0 |
| Working Example 41 | Active ingredient for hair 24 | | | | | | | |
| Working Example 42 | Active ingredient for hair 25 | Proline | $R^1$:$-CH_2-$, $R^2$:$-(CH_2)_2-$ or $R^1$:$-(CH_2)_2-$, $R^2$:$-CH_2-$ | | - | 1 | 1 | 0 |
| Working Example 43 | Active ingredient for hair 26 | γ-aminobutyric acid | - | H, H | $CH_2CH_2$ | 0 | 2 | 1 |
| Working Example 44 | Active ingredient for hair 27 | Serine | - | H, $CH_2OH$ | - | 0 | 2 | 0 |
| Comparative Example 1 | Active ingredient for hair 8 | L-Arginine | - | H,$(CH_2)_3NHC(NH)NH_2$ | - | 0 | 2 | 0 |

(continued)

| | Active ingredient for hair | Component (A) | | | | | | |
|---|---|---|---|---|---|---|---|---|
| | | Compound | $R^1$ | $R^2$ | $R^3$ | l | m | n |
| Comparative Example 2 | Active ingredient for hair 9 | Tromethamine | - | - | - | - | - | - |
| Comparative Example 3 | Active ingredient for hair 10 | - | - | - | - | - | - | - |
| Comparative Example 4 | Active ingredient for hair 11 | - | - | - | - | - | - | - |
| Comparative Example 5 | - | - | | | | | | |

[Table 1B]

| | Active ingredient for hair | Component (B) | | Compounding molar ratio (Component (A): Component (B)) | Molecular weight of active ingredient for hair |
|---|---|---|---|---|---|
| | | Type | Compound | | |
| Working Example 1 | Active ingredient for hair 1 | Saturated aliphatic monocarboxylic acid | Acetic acid | 0.5:1 | 294.3 |
| Working Example 2 | Active ingredient for hair 2 | Saturated aliphatic hydroxy monocarboxylic acid | Lactic acid | 0.5:1 | 354.4 |
| Working Example 3 | Active ingredient for hair 3 | Saturated aliphatic hydroxy di- or tricarboxylic acid | L-Malic acid | 1:1 | 308.3 |
| Working Example 4 | Active ingredient for hair 4 | | Tartaric acid | 1:1 | 324.3 |
| Working Example 5 | Active ingredient for hair 5 | | Citric acid | 1:1 | 366.3 |
| Working Example 6 | Active ingredient for hair 6 | | | 1.5:1 | 906.8 |
| Working Example 7 | Active ingredient for hair 7 | Saturated aliphatic hydroxy monocarboxylic acid | Lactic acid | 1:1 | 211.2 |
| Working Example 40 | Active ingredient for hair 23 | Saturated aliphatic hydroxy monocarboxylic acid | Lactic acid | 0.5:1 | 335.3 |
| Working Example 41 | Active ingredient for hair 24 | Saturated aliphatic hydroxy di-or tricarboxylic acid | Citric acid | 1.5:1 | 849.7 |
| Working Example 42 | Active ingredient for hair 25 | Saturated aliphatic hydroxy monocarboxylic acid | Lactic acid | 1:1 | 205.2 |
| Working Example 43 | Active ingredient for hair 26 | Saturated aliphatic hydroxy monocarboxylic acid | Lactic acid | 1:1 | 193.2 |
| Working Example 44 | Active ingredient for hair 27 | Saturated aliphatic hydroxy monocarboxylic acid | Lactic acid | 1:1 | 195.2 |
| Comparative Example 1 | Active ingredient for hair 8 | - | - | - | 174.2 |
| Comparative Example 2 | Active ingredient for hair 9 | - | - | - | 121.1 |

(continued)

| | Active ingredient for hair | Component (B) | | Compounding molar ratio (Component (A): Component (B)) | Molecular weight of active ingredient for hair |
|---|---|---|---|---|---|
| | | Type | Compound | | |
| Comparative Example 3 | Active ingredient for hair 10 | Saturated aliphatic hydroxy monocarboxylic acid | Lactic acid | - | 90.1 |
| Comparative Example 4 | Active ingredient for hair 11 | Saturated aliphatic hydroxy di-or tricarboxylic acid | L-Malic acid | - | 134.1 |
| Comparative Example 5 | - | - | | - | - |

[Table 1C]

| | Active ingredient for hair | State at *25°C* | Added concentration (wt%) | Sensory evaluation A | | | | |
|---|---|---|---|---|---|---|---|---|
| | | | | Easticity/ resilience | Non-waviness | Finger-combing capability | Moist feeling | Cohesive-ness |
| Working Example 1 | Active ingredient for hair 1 | Solid | 1 | ○ | ○ | ○ | ○ | ○ |
| Working Example 2 | Active ingredient for hair 2 | Liquid | 1 | ◎ | ◎ | ◎ | ◎ | ◎ |
| Working Example 3 | Active ingredient for hair 3 | Liquid | 1 | ◎ | ◎ | ◎ | ◎ | ◎ |
| Working Example 4 | Active ingredient for hair 4 | Liquid | 1 | ◎ | ◎ | ◎ | ◎ | ◎ |
| Working Example 5 | Active ingredient for hair 5 | Liquid | 1 | ◎ | ◎ | ◎ | ◎ | ◎ |
| Working Example 6 | Active ingredient for hair 6 | Liquid | 1 | ◎ | ◎ | ◎ | ◎ | ◎ |
| Working Example 7 | Active ingredient for hair 7 | Liquid | 1 | ○ | ◎ | ○ | ◎ | ◎ |
| Working Example 40 | Active ingredient for hair 23 | Liquid | 1 | ◎ | ◎ | ◎ | ◎ | ◎ |
| Working Example 41 | Active ingredient for hair 24 | Liquid | 1 | ◎ | ◎ | ◎ | ◎ | ◎ |
| Working Example 42 | Active ingredient for hair 25 | Liquid | 1 | ◎ | ◎ | ◎ | ◎ | ◎ |

(continued)

| | Active ingredient for hair | State at 25°C | Added concentration (wt%) | Sensory evaluation A | | | | |
|---|---|---|---|---|---|---|---|---|
| | | | | Easticity/ resilience | Non-waviness | Finger-combing capability | Moist feeling | Cohesive-ness |
| Working Example 43 | Active ingredient for hair 26 | Liquid | 1 | ◎ | ◎ | ◎ | ◎ | ◎ |
| Working Example 44 | Active ingredient for hair 27 | Liquid | 1 | ◎ | ◎ | ◎ | ◎ | ◎ |
| Comparative Example 1 | Active ingredient for hair 8 | Solid | 1 | Δ | Δ | × | Δ | × |
| Comparative Example 2 | Active ingredient for hair 9 | Solid | 1 | Δ | Δ | × | Δ | × |
| Comparative Example 3 | Active ingredient for hair 10 | Liquid | 1 | Δ | × | Δ | Δ | Δ |
| Comparative Example 4 | Active ingredient for hair 11 | Solid | 1 | Δ | × | Δ | Δ | Δ |
| Comparative Example 5 | - | - | - | × | × | × | × | × |

The results in Tables 1A to 1C demonstrated that the active ingredients for hair 1 to 7 and 23 to 27 of the working examples were excellent for the evaluations of elasticity/resilience, non-waviness, finger-combing capability, moist feeling, and cohesiveness after being air dried compared to those of the comparative examples, thus exhibiting enhanced efficacy in repairing hair.

The active ingredient for hair of the present invention exhibited the efficacy of permeating into hair to repair the hair and the treatment efficacy of repairing the surface of the hair as a useful component, thereby suggesting its high utility.

Comparing Working Example 1 and Working Examples 2 to 6, where the component (A) was arginine, the examples in which the component (B) was a saturated aliphatic hydroxy monocarboxylic acid or a saturated aliphatic hydroxy di- or tricarboxylic acid resulted in more favorable tactile sensations than the one in which the component (B) was a saturated aliphatic monocarboxylic acid.

Meanwhile, comparing Working Example 2 and Working Examples 7, 40, and 42 to 44 from each other, where the component (B) was lactic acid, Working Examples 2, 40, and 42 to 44 in which the component (A) was an amino acid resulted in favorable tactile sensations.

Working Examples 2 to 6 and 40 to 44 that were liquid at 25°C provided more favorable tactile sensations than Working Example 1 that was solid at 25°C.

1-2 Evaluation of hair repairment efficacy (cuticle repairment efficacy) by hair surface observation using scanning electron microscope

A scanning electron microscope (SEM by Hitachi High-Tech Corporation, S-3400N) was used to observe the surface conditions of hairs treated in accordance with 1-1. FIG. 1 shows a SEM image of Comparative Example 5, while FIG. 2 shows a SEM image of Working Example 3.

These SEM images show that the active ingredient for hair worked on the hair to suppress cuticles from being raised, thus demonstrating its efficacy in repairing the hair's surface. Consequently, it can be inferred that finger-combing capability, moisture retention, and cohesiveness were improved.

2. Permeability evaluation of hair additive in composition for hair

Damaged hairs were prepared using the operations as described above. Then, 1 g of each of the active ingredients 1 to 7 and 12 for hair and 5 g of succinic acid (manufactured by KANTO CHEMICAL CO., INC.) were dissolved in 94 g of a 50% by mass ethanol aqueous solution to prepare respective samples labeled as Working Examples 8 to 15. In addition, 5 g of succinic acid was dissolved in 95 g of a 50% by mass ethanol aqueous solution to prepare a sample labeled as Comparative Example 6. 1 g of damaged hair was immersed for 15 minutes in each sample, after which it was rinsed with running water for 30 seconds, dried with a towel and then subjected to air drying to make a post-treatment hair. The treated hair was shredded and then subjected to ethanol extraction to isolate the succinic acid that had permeated into the hair, and the amount of succinic acid that had permeated into the hair ($\mu$g: succinic acid/g: hair) was quantified using ion chromatography (Dionex Integration HPIC, manufactured by Thermo Fisher Scientific). Comparative Example 6 was used as a reference (100%) to calculate the respective permeability percentage.

The permeability percentages of the respective working examples were calculated using a reference of Comparative Example 6 as being 100. (Permeability percentage (%) = (Permeated amount of each working example/-Permeated amount of Comparative Example 6) $\times$ 100)

[Table 2A]

| | Active ingredient for hair | Component (A) | | | | | | |
|---|---|---|---|---|---|---|---|---|
| | | Compound | $R^1$ | $R^2$ | $R^3$ | l | m | n |
| Working Example 8 | Active ingredient for hair 1 | Arginine | - | H, $(CH_2)_3NHC(NH)NH_2$ | - | 0 | 2 | 0 |
| Working Example 9 | Active ingredient for hair 2 | | | | | | | |
| Working Example 10 | Active ingredient for hair 12 | | | | | | | |
| Working Example 11 | Active ingredient for hair 3 | | | | | | | |
| Working Example 12 | Active ingredient for hair 4 | | | | | | | |
| Working Example 13 | Active ingredient for hair 5 | | | | | | | |
| Working Example 14 | Active ingredient for hair 6 | | | | | | | |
| Working Example 15 | Active ingredient for hair 7 | Tromethamine | - | - | - | - | - | - |
| Comparative Example 6 | - | - | | | | | | |

[Table 2B]

| | Active ingredient for hair | Component (B) | | Compounding molar ratio (Component (A): Component (B)) | Molecular weight of active ingredient for hair | State at 25°C | Perme-ability percent-age (%) |
|---|---|---|---|---|---|---|---|
| | | Type | Compound | | | | |
| Working Example 8 | Active ingredient for hair 1 | Saturated aliphatic monocarboxylic acid | Acetic acid | 0.5:1 | 294 | Solid | 105 |

(continued)

| | Active ingredient for hair | Component (B) | | Compounding molar ratio (Component (A): Component (B)) | Molecular weight of active ingredient for hair | State at 25°C | Perme -ability percent -age (%) |
|---|---|---|---|---|---|---|---|
| | | Type | Compound | | | | |
| Working Example 9 | Active ingredient for hair 2 | Saturated aliphatic hydroxy monocarboxylic acid | Lactic acid | 0.5:1 | 354 | Liquid | 123 |
| Working Example 10 | Active ingredient for hair 12 | | Gluconic acid | 0.5:1 | 567 | Liquid | 121 |
| Working Example 11 | Active ingredient for hair 3 | Saturated aliphatic hydroxy di-or tricarboxylic acid | Malic acid | 1:1 | 308 | Liquid | 112 |
| Working Example 12 | Active ingredient for hair 4 | | Tartaric acid | 1:1 | 324 | Liquid | 110 |
| Working Example 13 | Active ingredient for hair 5 | | Citric acid | 1:1 | 366 | Liquid | 119 |
| Working Example 14 | Active ingredient for hair 6 | | | 1.5:1 | 907 | Liquid | 119 |
| Working Example 15 | Active ingredient for hair 7 | Saturated aliphatic hydroxy monocarboxylic acid | Lactic acid | 1:1 | 211 | Liquid | 120 |
| Comparative Example 6 | - | | | - | - | - | 100 |

The results in Tables 2A and 2B show that the permeability percentages of Working Examples 8 to 15 (active ingredients for hair 1 to 7 and 12) were greater than that of Comparative Example 6, demonstrating the superior permeability of the hair additive in the composition for hair of the present invention consisting of the component (A) and the component (B).

In other words, it was suggested that the active ingredient for hair of the present invention has excellent solubility of hair additives and permeability efficacy, and is highly useful as an active ingredient for hair. Furthermore, it fully brought out the effects of astringents such as succinic acid, making it suitable for use in repairing both the surface and interior regions of hair.

When comparing Working Example 8 and Working Examples 9 to 14, which have the same component (A), the one in which the component (B) was a saturated aliphatic hydroxy monocarboxylic acid or a saturated aliphatic hydroxy di- or tricarboxylic acid showed better permeability than the one in which the component (B) was a saturated aliphatic monocarboxylic acid.

Meanwhile, when comparing Working Example 9 and Working Example 15, where component (B) was lactic acid, Working Example 9, where component (A) was an amino acid, exhibited better permeability. Furthermore, Working Examples 9 to 14, which were liquids at 25°C, imparted better permeability compared to Working Example 8 which was a solid at 25°C.

3. Permeability evaluation of active ingredient for hair and hair additive in composition for hair

Damaged hairs subjected to the bleach treatment were used to perform permeability examination of the active ingredient for hair and the hair additive. Damaged hairs were prepared using the operation as described above. Next, 1 g of each active ingredient for hair and 0.3 g of blue dye (ACID Violet 43, manufactured by Combi-Blocks Inc.) were dissolved in

98.7 g of 50% by mass aqueous ethanol solution to prepare colorants respectively labeled as Working Examples 16 to 28 and Comparative Examples 7 to 14. Also, 0.3 g of blue dye (ACID Violet 43, manufactured by Combi-Blocks Inc.) was dissolved in 99.7 g of 50% by mass aqueous ethanol solution to prepare a colorant labeled as Comparative Example 15. Next, 1 g of damaged white hair was immersed in each colorant for 15 minutes, then rinsed with running water for 30 seconds, towel-dried and then air-dried to obtain a dyed hair sample A.

3-1. Permeability evaluation by hair cross sectional observation using optical microscope observation

Thin hair sections were prepared from the dyed hair sample A using a microtome, and the cross-sections of the dyed hair samples were observed using an optical microscope (Axio Imager manufactured by ZEISS., A2m). FIGs. 3A, 4A, 5A, 6A, and 7A respectively show optical microscope pictures of the hairs, according to Working Examples 17, 18, and 24 and Comparative Examples 7 and 15. To evaluate the permeability of the dye, the micrographs of the FIGs. 3A to 7A were taken into an image processing tool of Photoshop® (manufactured by Adobe Inc.), and the areas, dyed in blue by the above hair dyeing procedure, were extracted by a color range as specified below and were shown in FIGs. 3B to 7B (the blue dyed area in FIGs. 3B to 7B were shown in black).

The color range in FIG. 3A to 6A was RGB = (0,175,175) to (175,0,175) which was in blue spectrum. FIG. 7B had very weak color and its colored hair region could not be extracted by the above-mentioned color range (image selection failed). For this reason, the RGB color range was specified as RGB = (50,50,100) and the tolerance (F) was set at 100.

Similar operations were also performed on Working Examples 16, 19 to 23, and 25 to 28, and Comparative Examples 8 to 14 to evaluate the permeability of the active ingredients for hair and the hair additive based on the optical microscope observation and the processed images using the criteria indicated below, and the results are as shown in Tables 3A and 3B.

<Permeability criteria>

◎ was assigned for those that permeated very well
○ was assigned for those that permeated well
△ was assigned for those that permeated slightly
× was assigned for those adhered on the surface.

The cross-sectional microscope images of dyed hair samples reveal that Working Examples 16 to 28 achieved deeper hair interior coloring of the blue dye compared to Comparative Examples 7 to 15. It was therefore suggested that the active ingredient for hair of the present invention and the hair additive were permeated into the hair. No further evaluations by the color difference and fixation were carried out for the comparative examples because they were inferior in terms of permeability.

The results of permeability in Tables 3A and 3B show that, among Working Examples 16 to 27 in which the component (A) was arginine, the one in which the component (B) was a saturated aliphatic hydroxy monocarboxylic acid or a saturated aliphatic hydroxy di- or tricarboxylic acid, particularly a saturated aliphatic hydroxy di- or tricarboxylic acid, demonstrated a superior permeability.

Further, Working Examples 17, 18, 22 to 25 in which the active ingredients for hair were liquid at 25°C exhibited superior permeability than Working Examples 16, 19 to 21, 26, 27 in which they were solid at 25°C, thus demonstrating superior efficacy of the active ingredient for hair being liquid at 25°C in terms of permeability of the active ingredient for hair and the hair additive.

3-2 Permeability Evaluation by color difference

The color difference of dyed hair sample A, before and after the coloring treatment, was determined as follows. A spectrophotometer (SD 7000, manufactured by NIPPON DENSHOKU INDUSTRIES CO., LTD.) was used to measure the hue ($a^*_1$, $b^*_1$) and luminosity ($L^*_1$) of the damaged hair prior to coloration. The hue ($a^*_2$, $b^*_2$) and luminosity ($L^*_1$) of the dyed hair sample A were measured in the same manner. The obtained measurement results were then used in the following formula 1 to calculate the color difference A ($\Delta E^*_{ab1}$).
[Mathematical formula 1]

$$\text{Color Difference A: } \Delta E^*_{ab1} = \sqrt{(L^*_2 - L^*_1)^2 + (a^*_2 - a^*_1)^2 + (b^*_2 - b^*_1)^2} \quad \cdots \text{Formula 1}$$

The following criteria were used to evaluate the color difference

<Criteria for color difference A >

| | |
|---|---|
| ◎ ($\Delta E^*_{ab1}$ of 35 or more): | Very strong permeation |
| O ($\Delta E^*_{ab1}$ of 30 or more and less than 35): | Strong permeation |
| Δ ($\Delta E^*_{ab1}$ of 20 or more and less than 30): | Moderate permeation |

The compositions for hair containing the active ingredients for hair 1 to 7 and 12 to 17 of Working Examples 16 to 28 permeated the hair additives favorably and achieved hair coloration.

Of the working examples, these color differences A demonstrate that, among Working Examples 16 to 27 in which the component (A) was arginine, the one in which the component (B) was a saturated aliphatic hydroxy monocarboxylic acid or a saturated aliphatic hydroxy di-or tricarboxylic acid, particularly a saturated aliphatic hydroxy di- or tricarboxylic acid, demonstrated a superior permeability.

Further, Working Example 17, 18, and 22 to 25 in which the active ingredients for hair were liquid at 25°C exhibited superior permeability thanWorking Examples 16, 19 to 21, 26, and 27 in which they were solid at 25°C, thus indicating superior efficacy of the active ingredient for hair being liquid at 25°C in terms of permeability of the hair additive.

3-3. Fixation property evaluation by fixation percentage

Furthermore, the color difference B of the dyed hair sample A after washing was determined as follows. 20 g of: a pre-adjusted shampoo concentrate (SPAMIN SA manufactured by Miyoshi Oil & Fat Co., Ltd.) in the amount of 11.25 parts by mass; Amphorex CB-1 (manufactured by Miyoshi Oil & Fats Co., Ltd.) in the amount of 3.75 parts by mass; Catinal HC-200 (manufactured by Toho Chemical Industry Co., Ltd.) in the amount of 0.5 parts by mass; and citric acid (for use in pH adjustment: manufactured by Wako Pure Chemical Corporation in appropriate amount and the remainder of water) was diluted with 120 g of ion-exchanged water at 40°C, and the dyed hair sample A was put thereinto and rubbed with fingers for washing for 1 minute. Thereafter, the sample was subjected to a shampoo step one time in which the sample was placed in 250 ml of ion-exchanged water at 40°C and washed for 20 seconds, where this process was repeated twice, and then the sample was towel-dried and air-dried to prepare a dyed hair sample B. A spectrophotometer was used in the manner as described above to measure the hue ($a^*_3$, $b^*_3$) and luminosity ($L^*_3$) of the obtained dyed hair sample B after the shampoo step. The obtained measurement results were then used in the following formula 2 to calculate the color difference B ($\Delta E^*_{ab2}$).
[Mathematical formula 2]

$$\text{Color Difference B: } \Delta E^*_{ab2} = \sqrt{(L^*_3 - L^*_2)^2 + (a^*_3 - a^*_2)^2 + (b^*_3 - b^*_2)^2} \quad \cdots \text{Formula 2}$$

The fixation percentages A were calculated from the color differences A and B and compared with each other: (Fixation percentage A (%) = (Color difference A - Color difference B)/ Color difference A) × 100)

The following criteria were used to evaluate the fixation percentage A.

<Criteria for fixation percentage A>

| | |
|---|---|
| ◎ (87% ≦ Fixation percentage A): | Excellent fixation |
| ○ (85% ≦ Fixation percentage A <87%): | Good fixation |
| Δ (80% ≦ Fixation percentage A < 85%) : | Moderate fixation |

(continued)

× (Fixation percentage A < 80%): Weak fixation

The compositions for hair of Working Examples 16 to 28 containing the active ingredients for hair 1 to 7 and 12 to 17 favorably fixed the hair additive.

Among Working Examples 16 to 27 in which the component (A) was arginine, the one in which the component (B) was a saturated aliphatic hydroxy monocarboxylic acid or a saturated aliphatic hydroxy di- or tricarboxylic acid, particularly a saturated aliphatic hydroxy di- or tricarboxylic acid, demonstrated a superior property of fixing the hair additive.

Further, Working Example 17, 18, and 22 to 25 in which the active ingredients for hair were liquid at 25°C exhibited superior fixation property than Working Example 16, 19 to 21, 26, 27 in which they were solid at 25°C, thus indicating superior efficacy of the active ingredient for hair being liquid at 25°C in terms of fixation property of the hair additive.

It was also demonstrated that the dyes were favorably permeated and fixed therein when the dyes employed Basic Blue 12 (Tokyo Chemical Industry Co., Ltd.) or HC Blue No. 2 (Tokyo Chemical Industry Co., Ltd.) to carry out the examination.

[Table 3A]

| | Active ingredient for hair | Component (A) | | | | | | | Component (B) |
|---|---|---|---|---|---|---|---|---|---|
| | | Compound | $R^1$ | $R^2$ | $R^3$ | l | m | n | Type |
| Working Example 16 | Active ingredient for hair 1 | | | | | | | | Saturated aliphatic monocarboxylic acid |
| Working Example 17 | Active ingredient for hair 2 | | | | | | | | Saturated aliphatic hydroxy monocarboxylic acid |
| Working Example 18 | Active ingredient for hair 12 | | | | | | | | |
| Working Example 19 | Active ingredient for hair 13 | | | | | | | | Saturated aliphatic dicarboxylic acid |
| Working Example 20 | Active ingredient for hair 14 | | | | | | | | |
| Working Example 21 | Active ingredient for hair 15 | Arginine | - | H, $(CH_2)_3NHC(NH)NH_2$ | - | 0 | 2 | 0 | Unsaturated aliphatic dicarboxylic acid |
| Working Example 22 | Active ingredient for hair 3 | | | | | | | | Saturated aliphatic hydroxy di- or tricarboxylic acid |
| Working Example 23 | Active ingredient for hair 4 | | | | | | | | |
| Working Example 24 | Active ingredient for hair 5 | | | | | | | | |
| Working Example 25 | Active ingredient for hair 6 | | | | | | | | |
| Working Example 26 | Active ingredient for hair 16 | | | | | | | | Aromatic hydroxycarboxylic acid |

(continued)

| | Active ingredient for hair | Component (A) | | | | | | | Component (B) |
|---|---|---|---|---|---|---|---|---|---|
| | | Compound | $R^1$ | $R^2$ | $R^3$ | l | m | n | Type |
| Working Example 27 | Active ingredient for hair 17 | | | | | | | | lactone with hydroxy group |
| Working Example 28 | Active ingredient for hair 7 | Tromethamine | - | - | - | - | - | - | Saturated aliphatic hydroxy monocarboxylic acid |
| Comparative Example 7 | Active ingredient for hair 8 | Arginine | - | H, $(CH_2)_3NHC(NH)NH_2$ | - | 0 | 2 | 0 | - |
| Comparative Example 8 | Active ingredient for hair 9 | Tromethamine | - | - | - | - | - | - | - |
| Comparative Example 9 | Active ingredient for hair 10 | - | - | - | - | - | - | - | Saturated aliphatic hydroxy monocarboxylic acid |
| Comparative Example 10 | Active ingredient for hair 18 | Arginine | - | H, $(CH_2)_3NHC(NH)$ NH, | - | 0 | 2 | 0 | - |
| Comparative Example 11 | Active ingredient for hair 19 | Diethyl sebacate | | | | | | | |
| Comparative Example 12 | Active ingredient for hair 20 | Benzyl alcohol | | | | | | | |
| Comparative Example 13 | Active ingredient for hair 21 | Tetrabutylammonium bromide | | | | | | | |
| Comparative Example 14 | Active ingredient for hair 22 | 1-butyl-3-methylimidazolium hydroxide | | | | | | | |
| Comparative Example 15 | - | - | | | | | | | |

[Table 3B]

| | Active ingredient for hair | Component (B) | Compounding molar ratio (Component (A) : Component (B)) | Molecular weight of active ingredient for hair | State at 25°C | Permeability | Color difference (⊿E) | | | Fixation percentage A (%) | |
|---|---|---|---|---|---|---|---|---|---|---|---|
| | | Compound | | | | | Color difference A | | Color differe -nce B | | |
| Working Example 16 | Active ingredient for hair 1 | Acetic acid | 0.5:1 | 294 | Solid | △ | 29.8 | △ | 5.0 | 83.2 | △ |
| Working Example 17 | Active ingredient for hair 2 | Lactic acid | 0.5:1 | 354 | Liquid | O | 34.4 | ○ | 4.5 | 86.9 | ○ |
| Working Example 18 | Active ingredient for hair 12 | Gluconic acid | 0.5:1 | 567 | Liquid | ○ | 34.1 | O | 4.5 | 86.8 | ○ |
| Working Example 19 | Active ingredient for hair 13 | Adipic acid | 1:1 | 320 | Solid | △ | 24.7 | △ | 4.0 | 83.8 | Δ |
| Working Example 20 | Active ingredient for hair 14 | Succinic acid | 1:1 | 292 | Solid | △ | 27.9 | Δ | 4.5 | 83.7 | Δ |
| Working Example 21 | Active ingredient for hair 15 | Fumaric acid | 1:1 | 290 | Solid | △ | 29.7 | △ | 4.8 | 83.8 | Δ |
| Working Example 22 | Active ingredient for hair 3 | Malic acid | 1:1 | 308 | Liquid | ◎ | 36.1 | ◎ | 4.7 | 87.0 | ◎ |
| Working Example 23 | Active ingredient for hair 4 | Tartaric acid | 1:1 | 324 | Liquid | ◎ | 37.2 | ◎ | 4.5 | 87.9 | ◎ |
| Working Example 24 | Active ingredient for hair 5 | Citric acid | 1:1 | 366 | Liquid | ◎ | 46.4 | ◎ | 2.3 | 95.0 | ◎ |
| Working Example 25 | Active ingredient for hair 6 | | 1.5:1 | 907 | Liquid | ◎ | 36.5 | ◎ | 4.4 | 87.9 | ◎ |
| Working Example 26 | Active ingredient for hair 16 | Gallic acid | 0.5:1 | 514 | Solid | △ | 22.1 | △ | 4.3 | 80.7 | Δ |
| Working Example 27 | Active ingredient for hair 17 | Ascorbic acid | 0.5:1 | 526 | Solid | △ | 29.7 | △ | 5.1 | 82.7 | △ |
| Working Example 28 | Active ingredient for hair 7 | Lactic acid | 1:1 | 211 | Liquid | ○ | 33.3 | O | 4.5 | 86.5 | ○ |

(continued)

| | Active ingredient for hair | Component (B) | Compounding molar ratio (Component (A) : Component (B)) | Molecular weight of active ingredient for hair | State at 25°C | Permeability | Color difference (⊿E) | | | Fixation percentage A (%) | |
|---|---|---|---|---|---|---|---|---|---|---|---|
| | | Compound | | | | | Color difference A | | Color differe -nce B | | |
| Comparative Example 7 | Active ingredient for hair 8 | - | - | 174 | Solid | × | - | - | - | - | - |
| Comparative Example 8 | Active ingredient for hair 9 | - | - | 121 | Solid | × | - | - | - | - | - |
| Comparative Example 9 | Active ingredient for hair 10 | Lactic acid | - | 90 | Liquid | × | - | - | - | - | - |
| Comparative Example 10 | Active ingredient for hair 18 | Hydrochloric acid | 1:1 | 211 | Solid | × | - | - | - | - | - |
| Comparative Example 11 | Active ingredient for hair 19 | | - | 258 | Liquid | × | - | - | - | - | - |
| Comparative Example 12 | Active ingredient for hair 20 | | - | 108 | Liquid | × | - | - | - | - | - |
| Comparative Example 13 | Active ingredient for hair 21 | | - | 322 | Solid | × | - | - | - | - | - |
| Comparative Example 14 | Active ingredient for hair 22 | | - | 156 | Liquid | × | - | - | - | - | - |
| Comparative Example 15 | - | | - | - | - | × | - | - | - | - | - |

4. Permeability and fixation evaluations of hair additive containing oxidation dye in composition for hair

Bigen Speedy Color Cream 6 (Dark Brown), manufactured by Hoyu Co., Ltd., was used as a conventional hair dye containing an oxidative coloring agent. 1g of Agent 1, 1g of Agent 2, and 2g of each active ingredient for hair (5% by mass aqueous solution) were quickly and uniformly mixed, and the mixture was rubbed evenly into 1g of white hair (manufactured by KETABAYA) for 3 minutes, and then left to stand for 17 minutes. The hair was then rinsed with 40°C ion-exchanged water for 40 seconds, towel-dried, and air-dried to obtain a dyed hair sample C.

4-1 Permeability Evaluation by color difference

A spectrophotometer (SD 7000, manufactured by NIPPON DENSHOKU INDUSTRIES CO., LTD.) was used to measure the hue ($a^*_4$, $b^*_4$) and luminosity ($L^*_4$) of the white hair prior to coloration. Then, the hue ($a^*_5$, $b^*_5$) and luminosity ($L^*_5$) of the dyed hair sample C as obtained above were measured.

The obtained measurement results were then used in the following formula 3 to calculate the color difference C ($\Delta E^*_{ab3}$).

[Mathematical formula 3]

$$\text{Color Difference C}: \Delta E^*_{ab3} = \sqrt{(L^*_5 - L^*_4)^2 + (a^*_5 - a^*_4)^2 + (b^*_5 - b^*_4)^2} \quad \cdots \text{Formula 3}$$

<Criteria for color difference C >

| | |
|---|---|
| ◎ ($66 \leq \Delta E^*_{ab3}$): | Very strong permeation |
| ○ ($65 \leqq \Delta E^*_{ab3} < 66$): | Strong permeation |
| △ ($64 \leqq \Delta E^*_{ab3} < 65$): | Moderate permeation (Same level without additive) |
| × ($\Delta E^*_{ab3} < 64$): | Weak permeation |

The color differences C in Tables 4A to 4D indicate that Working Examples 29 to 39 and 45 to 49 showed larger color differences than Comparative Examples 16 to 19, thereby demonstrating excellent permeability. In other words, it was suggested that the hair additives were permeated in the hair.

Of the working examples, among Working Examples 29 to 38 and 45 to 49 in which the component (A) was an amino acid, the one in which the component (B) was a saturated aliphatic hydroxy monocarboxylic acid or a saturated aliphatic hydroxy di- or tricarboxylic acid demonstrated a superior permeability.

Further, Working Examples 30, 31, 34 to 37, and 45 to 49 in which the active ingredients for hair were liquid at 25°C exhibited superior permeability than Working Examples 29, 32, 33, and 38 in which they were solid at 25°C, thus indicating superior efficacy of the active ingredient for hair being liquid at 25°C in terms of permeability of the hair additive.

4-2. Evaluation on fixation property by fixation percentage

20 g of the above-mentioned shampoo concentrate was diluted with 120 g of ion-exchanged water at 40°C, and the dyed hair sample C was put thereinto, and scrubbed for washing for 1 minute. Then, the sample was washed with 250 mL of 40°C ion-exchanged water for 20 seconds (twice), and then the sample was towel-dried and air-dried. This process was repeated five times to obtain a dyed hair sample D. The hue ($a^*_6$, $b^*_6$) and luminosity ($L^*_6$) of the obtained dyed hair sample D were measured.

The obtained measurement results were used in the following formula 4 to calculate the color difference D ($\Delta E^*_{ab4}$).

[Mathematical formula 4]

$$\text{Color Difference D}: \Delta E^*_{ab4} = \sqrt{(L^*_6 - L^*_5)^2 + (a^*_6 - a^*_5)^2 + (b^*_6 - b^*_5)^2} \quad \cdots \text{Formula 4}$$

Further, the fixation percentages B were calculated from the color differences C and D and compared with each other: (Fixation percentage B (%) = (Color difference C - Color difference D)/ Color difference C) × 100)

The following criteria were used to evaluate the fixation percentage B.

<Criteria for fixation percentage B>

| | |
|---|---|
| ◎ (97% ≦ Fixation percentage B): | Excellent fixation |
| ○ (95% ≦ Fixation percentage B <97%): | Good fixation |
| △ (90% ≦ Fixation percentage B < 95%): | Moderate fixation |
| × (Fixation percentage B < 90%): | Weak fixation |

The results of the fixation percentages B in Tables 4A to 4D demonstrate that Working Examples 29 to 39 and 45 to 49 exhibited superior fixation properties compared to Comparative Examples 16, 18, and 19. These results suggest that the composition for hair, containing both the active ingredient of the present invention and the hair additive, exhibits an excellent property of fixing the hair additive when it is applied to hair.

Of the working examples, among Working Examples 29 to 38 and 45 to 49 in which the component (A) was an amino acid, the one in which the component (B) was a saturated aliphatic hydroxy di- or tricarboxylic acid demonstrated a superior fixation property.

It was further demonstrated that those having the compounding ratios between the components (A) and (B) of 0.005:1 and 20:1 were inferior to the one of Working Example 29 in terms of the color difference C and fixation percentage B.

1 g of white hair (manufactured by KETABAYA) was immersed in each active ingredient for hair (5% by mass aqueous solution) for 10 minutes, towel-dried, and then air-dried to obtain hairs treated with the respective active ingredient for hair. Next, Bigen Speedy Color Cream 6 (Dark Brown), manufactured by Hoyu Co., Ltd., was used as a hair dye to perform the hair coloring treatment. Specifically, 1g of Agent 1 and 1g of Agent 2 were quickly and uniformly mixed, and the mixture was rubbed evenly for 3 minutes into the hair treated with each active ingredient for hair, and then left to stand for 17 minutes. The mixture was then rinsed with 40°C ion-exchanged water for 40 seconds, towel-dried, and air-dried to obtain a dyed hair sample. The resultant dyed hair samples were evaluated by the same method as in 4-1 and 4-2 to calculate the color difference C and the fixation percentage B and carry out the sensory evaluation

As a result, similar to 4-1 and 4-2, the hair additive in the composition for hair (active ingredient + oxidative dye) demonstrated improvements in permeability, fixation property, hair repairment efficacy, and tactile sensation.

4-3. Evaluations on hair repairment efficacy (cuticle repairment efficacy) and tactile sensation by sensory evaluation

The dyed hair sample C was evaluated for the aspects of: elasticity/resilience, non-waviness, finger-combing capability, moist feeling, and cohesiveness from the perspective of superiority or inferiority to a test hair bundle uncoated with the sample.

Ten panel members were randomly selected regardless of age and sex for evaluation.

◎: Superior
O: Favorable
Δ: No change
×: Poor

The sensory evaluations in Tables 4A to 4D demonstrated that Working Examples 29 to 39, and 45 to 49 were excellent for every aspect of elasticity/resilience, non-waviness, finger-combing capability, moist feeling, and cohesiveness compared to those of Comparative Examples 16 to 19. These results suggest that the composition for hair, containing both the active ingredient of the present invention and the hair additive, exhibits excellent hair repair efficacy when it is applied to hair.

Among Working Examples 29 to 38 and 45 to 49 in which the component (A) was an amino acid, those using the component (B) as a saturated aliphatic hydroxy monocarboxylic acid or a saturated aliphatic hydroxy di- or tricarboxylic acid produced a more favorable tactile sensation.

Among Working Examples 30, 39, 45, and 47 to 49 in which the component (B) was lactic acid, Working Examples 30, 45, and 47 to 49 in which the component (A) was an amino acid produced a more favorable tactile sensation.

Further, Working Examples 30, 31, 34 to 37, and 45 to 49 that were liquid at 25°C provided a more favorable tactile

sensation than Working Examples 29, 32, 33, and 38 that were solid at 25°C.

[Table 4A]

| | Active ingredient for hair | Component (A) | | | | | | |
|---|---|---|---|---|---|---|---|---|
| | | Compound | $R^1$ | $R^2$ | $R^3$ | l | m | n |
| Working Example 29 | Active ingredient for hair 1 | L-Arginine | - | H, $(CH_2)_3NHC(NH)NH_2$ | - | 0 | 2 | 0 |
| Working Example 30 | Active ingredient for hair 2 | | | | | | | |
| Working Example 31 | Active ingredient for hair 12 | | | | | | | |
| Working Example 32 | Active ingredient for hair 13 | | | | | | | |
| Working Example 33 | Active ingredient for hair 15 | | | | | | | |
| Working Example 34 | Active ingredient for hair 3 | | | | | | | |
| Working Example 35 | Active ingredient for hair 4 | | | | | | | |
| Working Example 36 | Active ingredient for hair 5 | | | | | | | |
| Working Example 37 | Active ingredient for hair 6 | | | | | | | |
| Working Example 38 | Active ingredient for hair 17 | | | | | | | |
| Working Example 39 | Active ingredient for hair 7 | Tromethamine | - | - | - | - | - | - |
| Working Example 45 | Active ingredient for hair 23 | L-Histidine | - | H, $CH_2$(1H-imidazol-4-yl) | - | 0 | 2 | 0 |
| Working Example 46 | Active ingredient for hair 24 | | | | | | | |
| Working Example 47 | Active ingredient for hair 25 | Proline | $R^1$:$-CH_2-$, $R^2$:$-(CH_2)_2-$or $R^1$:$-(CH_2)_2-$, $R^2$:$-CH_2-$ | | - | 1 | 1 | 0 |
| Working Example 48 | Active ingredient for hair 26 | γ-aminobutyric acid | - | H, H | $CH_2CH_2$ | 0 | 2 | 1 |
| Working Example 49 | Active ingredient for hair 27 | Serine | - | H, $CH_2OH$ | - | 0 | 2 | 0 |
| Comparative Example 16 | Active ingredient for hair 8 | L-Arginine | - | H,$(CH_2)_3NHC(NH)NH_2$ | - | 0 | 2 | 0 |

(continued)

| | Active ingredient for hair | Component (A) | | | | | | |
|---|---|---|---|---|---|---|---|---|
| | | Compound | $R^1$ | $R^2$ | $R^3$ | l | m | n |
| Comparative Example 17 | Active ingredient for hair 10 | - | - | - | - | - | - | - |
| Comparative Example 18 | Active ingredient for hair 18 | L-Arginine | - | H,$(CH_2)_3NHC(NH)NH_2$ | - | 0 | 2 | 0 |
| Comparative Example 19 | - | - | | | | | | |

[Table 4B]

| | Active ingredient for hair | Component (B) | | Compounding molar ratio (Component (A): Component (B)) | Molecular weight of active ingredient for hair |
|---|---|---|---|---|---|
| | | Type | Compound | | |
| Working Example 29 | Active ingredient for hair 1 | Saturated aliphatic monocarboxylic acid | Acetic acid | 0.5:1 | 294.3 |
| Working Example 30 | Active ingredient for hair 2 | Saturated aliphatic hydroxy monocarboxylic acid | Lactic acid | 0.5:1 | 354.4 |
| Working Example 31 | Active ingredient for hair 12 | | Gluconic acid | 0.5:1 | 566.5 |
| Working Example 32 | Active ingredient for hair 13 | Saturated aliphatic dicarboxylic acid | Adipic acid | 1:1 | 320.3 |
| Working Example 33 | Active ingredient for hair 15 | Unsaturated aliphatic dicarboxylic acid | Fumaric acid | 1:1 | 290.3 |
| Working Example 34 | Active ingredient for hair 3 | Saturated aliphatic hydroxy di-or tricarboxylic acid | L-Malic acid | 1:1 | 308.3 |
| Working Example 35 | Active ingredient for hair 4 | | Tartaric acid | 1:1 | 324.3 |
| Working Example 36 | Active ingredient for hair 5 | | Citric acid | 1:1 | 366.3 |
| Working Example 37 | Active ingredient for hair 6 | | | 1.5:1 | 906.8 |
| Working Example 38 | Active ingredient for hair 17 | lactone with hydroxy group | Ascorbic acid | 0.5:1 | 526.4 |
| Working Example 39 | Active ingredient for hair 7 | Saturated aliphatic hydroxy monocarboxylic acid | Lactic acid | 1:1 | 211.2 |
| Working Example 45 | Active ingredient for hair 23 | Saturated aliphatic hydroxy monocarboxylic acid | Lactic acid | 0.5:1 | 335.3 |

(continued)

| | Active ingredient for hair | Component (B) | | Compounding molar ratio (Component (A): Component (B)) | Molecular weight of active ingredient for hair |
|---|---|---|---|---|---|
| | | Type | Compound | | |
| Working Example 46 | Active ingredient for hair 24 | Saturated aliphatic hydroxy di-or tricarboxylic acid | Citric acid | 1.5:1 | 849.7 |
| Working Example 47 | Active ingredient for hair 25 | Saturated aliphatic hydroxy monocarboxylic acid | Lactic acid | 1:1 | 205.2 |
| Working Example 48 | Active ingredient for hair 26 | Saturated aliphatic hydroxy monocarboxylic acid | Lactic acid | 1:1 | 193.2 |
| Working Example 49 | Active ingredient for hair 27 | Saturated aliphatic hydroxy monocarboxylic acid | Lactic acid | 1:1 | 195.2 |
| Comparative Example 16 | Active ingredient for hair 8 | - | - | - | 174.2 |
| Comparative Example 17 | Active ingredient for hair 10 | Saturated aliphatic hydroxy monocarboxylic acid | Lactic acid | - | 90.1 |
| Comparative Example 18 | Active ingredient for hair 18 | - | Hydrochloric acid | - | 210.7 |
| Comparative Example 19 | - | - | | - | - |

[Table 4C]

| | Active ingredient for hair | State at 25°C | Color difference (⊿E) | | | Fixation percentage B (%) | |
|---|---|---|---|---|---|---|---|
| | | | Color difference C | | Color difference D | | |
| Working Example 29 | Active ingredient for hair 1 | Solid | 64.9 | Δ | 2.1 | 96.7 | ○ |
| Working Example 30 | Active ingredient for hair 2 | Liquid | 65.6 | ○ | 2.8 | 95.7 | ○ |
| Working Example 31 | Active ingredient for hair 12 | Liquid | 65.7 | ○ | 2.9 | 95.6 | ○ |
| Working Example 32 | Active ingredient for hair 13 | Solid | 64.8 | △ | 2.5 | 96.1 | ○ |
| Working Example 33 | Active ingredient for hair 15 | Solid | 64.9 | Δ | 2.5 | 96.1 | ○ |
| Working Example 34 | Active ingredient for hair 3 | Liquid | 66.2 | ◎ | 1.8 | 97.3 | ◎ |
| Working Example 35 | Active ingredient for hair 4 | Liquid | 66.1 | ◎ | 1.6 | 97.6 | ◎ |
| Working Example 36 | Active ingredient for hair 5 | Liquid | 66.0 | ◎ | 1.8 | 97.2 | ◎ |

(continued)

| | Active ingredient for hair | State at 25°C | Color difference (⊿E) | | | Fixation percentage B (%) | |
|---|---|---|---|---|---|---|---|
| | | | Color difference C | | Color difference D | | |
| Working Example 37 | Active ingredient for hair 6 | Liquid | 66.0 | ◎ | 1.9 | 97.1 | ◎ |
| Working Example 38 | Active ingredient for hair 17 | Solid | 64.9 | Δ | 2.5 | 96.2 | ○ |
| Working Example 39 | Active ingredient for hair 7 | Liquid | 65.2 | ○ | 2.9 | 95.6 | ○ |
| Working Example 45 | Active ingredient for hair 23 | Liquid | 66.4 | ◎ | 1.1 | 98.3 | ◎ |
| Working Example 46 | Active ingredient for hair 24 | Liquid | 66.1 | ◎ | 1.7 | 97.4 | ◎ |
| Working Example 47 | Active ingredient for hair 25 | Liquid | 66.0 | ◎ | 1.7 | 97.4 | ◎ |
| Working Example 48 | Active ingredient for hair 26 | Liquid | 66.0 | ◎ | 2.0 | 97.0 | ◎ |
| Working Example 49 | Active ingredient for hair 27 | Liquid | 66.2 | ◎ | 2.0 | 97.0 | ◎ |
| Comparative Example 16 | Active ingredient for hair 8 | Solid | 63.3 | × | 6.8 | 89.3 | × |
| Comparative Example 17 | Active ingredient for hair 10 | Liquid | 57.9 | × | - | - | - |
| Comparative Example 18 | Active ingredient for hair 18 | Solid | 64.1 | Δ | 5.0 | 92.2 | Δ |
| Comparative Example 19 | - | - | 64.7 | Δ | 4.8 | 92.6 | Δ |

[Table 4D]

| | Active ingredient for hair | Sensory evaluation B | | | | |
|---|---|---|---|---|---|---|
| | | Easticity /resilience | Non-waviness | Finger-combing capability | Moist feeling | Cohesiveness |
| Working Example 29 | Active ingredient for hair 1 | ○ | ○ | ○ | ○ | ○ |
| Working Example 30 | Active ingredient for hair 2 | ◎ | ◎ | ◎ | ◎ | ◎ |
| Working Example 31 | Active ingredient for hair 12 | ◎ | ◎ | ◎ | ◎ | ◎ |
| Working Example 32 | Active ingredient for hair 13 | ○ | ○ | ○ | ○ | ○ |
| Working Example 33 | Active ingredient for hair 15 | ○ | ○ | ○ | ○ | ○ |
| Working Example 34 | Active ingredient for hair 3 | ◎ | ◎ | ◎ | ◎ | ◎ |

(continued)

| | Active ingredient for hair | Sensory evaluation B | | | | |
|---|---|---|---|---|---|---|
| | | Easticity /resilience | Non-waviness | Finger-combing capability | Moist feeling | Cohesiveness |
| Working Example 35 | Active ingredient for hair 4 | ◎ | ◎ | ◎ | ◎ | ◎ |
| Working Example 36 | Active ingredient for hair 5 | ◎ | ◎ | ◎ | ◎ | ◎ |
| Working Example 37 | Active ingredient for hair 6 | ◎ | ◎ | ◎ | ◎ | ◎ |
| Working Example 38 | Active ingredient for hair 17 | ○ | ○ | ○ | ○ | ○ |
| Working Example 39 | Active ingredient for hair 7 | ◎ | ◎ | ○ | ◎ | ◎ |
| Working Example 45 | Active ingredient for hair 23 | ◎ | ◎ | ◎ | ◎ | ◎ |
| Working Example 46 | Active ingredient for hair 24 | ◎ | ◎ | ◎ | ◎ | ◎ |
| Working Example 47 | Active ingredient for hair 25 | ◎ | ◎ | ◎ | ◎ | ◎ |
| Working Example 48 | Active ingredient for hair 26 | ◎ | ◎ | ◎ | ◎ | ◎ |
| Working Example 49 | Active ingredient for hair 27 | ◎ | ◎ | ◎ | ◎ | ◎ |
| Comparative Example 16 | Active ingredient for hair 8 | Δ | Δ | ð. | △ | △ |
| Comparative Example 17 | Active ingredient for hair 10 | Δ | Δ | Δ | Δ | Δ |
| Comparative Example 18 | Active ingredient for hair 18 | Δ | Δ | Δ | Δ | Δ |
| Comparative Example 19 | - | Δ | Δ | Δ | Δ | Δ |

Accordingly, when the active ingredient for hair of the present invention is incorporated into a hair treatment agent, it exhibits an excellent oxidative dye fixation effect, thereby enhancing hair dyeing efficacy, suppressing color fading, and improving usability-including hair repairment and damage inhibition. These findings suggest that the active ingredient for hair of the present invention is highly beneficial for use in hair treatment agents.

## Claims

1. An active ingredient for hair comprising components (A) and (B) of:

   (A) an amine or ammonium compound having a hydrogen-bonding functional group; and
   (B) a carboxylic acid or a salt thereof,

   wherein the ingredient contains the components (A) and (B) at a molar ratio from 0.01:1 to 10:1.

2. The active ingredient for hair according to claim 1, wherein the component (A) is an amino acid represented by formula (1) or a salt thereof, the formula (1) being defined as

[Chemical formula 1]

$$R'_lNH_mC(R^2)_2(R^3{}_nCOOX) \qquad (1)$$

wherein each $R^1$ represents a monovalent or bivalent hydrocarbon group having 1 to 22 carbon atoms, each $R^2$ independently represents a hydrogen atom, a monovalent or bivalent hydrocarbon group having 1 to 22 carbon atoms, a monovalent or bivalent nitrogen-containing group, a monovalent or bivalent oxygen-containing group, or a monovalent or bivalent sulfur-containing group, $R^3$ represents a bivalent hydrocarbon group having 1 to 22 carbon atoms, 1 is a integer of 0 to 2 and m is a integer of 0 to 2 provided that the sum of 1 and m is 2, and n is 0 or 1; wherein $R^1$ and $R^2$ together optionally form a ring having 3 to 22 carbon atoms; and wherein X represents a hydrogen atom or a monovalent cation.

**3.** The active ingredient for hair according to claim 2, wherein the amino acid is an amino acid (a) in which a ratio of the total number of primary or secondary amino groups to the number of carboxy groups (the total number of primary or secondary amino groups/ the number of carboxy groups) is greater than 1.

**4.** The active ingredient for hair according to claim 1, wherein the component (A) is an amine compound represented by formula (2) defined as
[Chemical formula 2]

$$N(H)_x(R^4)_y(R^5)_z \qquad (2)$$

wherein each $R^4$ independently represents a hydrocarbon group having at least one hydroxy group, each $R^5$ independently represents a hydrocarbon group having 1 to 22 carbon atoms, y is an integer of 1 to 3 and each of x and z is an integer of 0 to 2 provided that the sum of x, y, and z is 3.

**5.** The active ingredient for hair according to claim 1, wherein the component (B) is a carboxylic acid or a salt thereof having a hydrogen-bonding functional group.

**6.** The active ingredient for hair according to claim 1, wherein the components (A) and (B) forms a mixture that is liquid at 25°C.

**7.** The active ingredient for hair according to claim 1, comprising an organic ammonium salt formed by the components (A) and (B).

**8.** The active ingredient for hair according to claim 7, wherein the organic ammonium salt is liquid at 25°C.

**9.** The active ingredient for hair according to any one of claims 1 to 8, wherein the ingredient is for use in a fixative agent for hair.

**10.** A composition for hair comprising: the active ingredient for hair according to claim 1; and a hair additive.

**11.** The composition for hair according to claim 10, wherein the hair additive is at least one additive selected from a dye, a coloring agent, a pigment, an astringent, and a reducing agent.

**12.** The composition for hair according to claim 10, comprising the hair additive and the active ingredient for hair at a compounding ratio of 10,000:1 to 1:10,000 by mass.

**13.** A method for coloring hair and/or suppressing color deterioration of colored hair, comprising the steps of:

applying the composition for hair according to claim 11, containing a dye as a hair additive, to hair; and
leaving the hair with the applied composition for a predetermined duration.

**14.** A method for coloring hair and/or suppressing color deterioration of colored hair, comprising:

a pretreatment step of applying a composition, containing the active ingredient for hair according to any one of claims 1 to 8, to hair; and
a hair coloring step of applying a composition, containing a dye, to the hair after the pretreatment step, and then

leaving the hair for a predetermined duration.

FIG.1

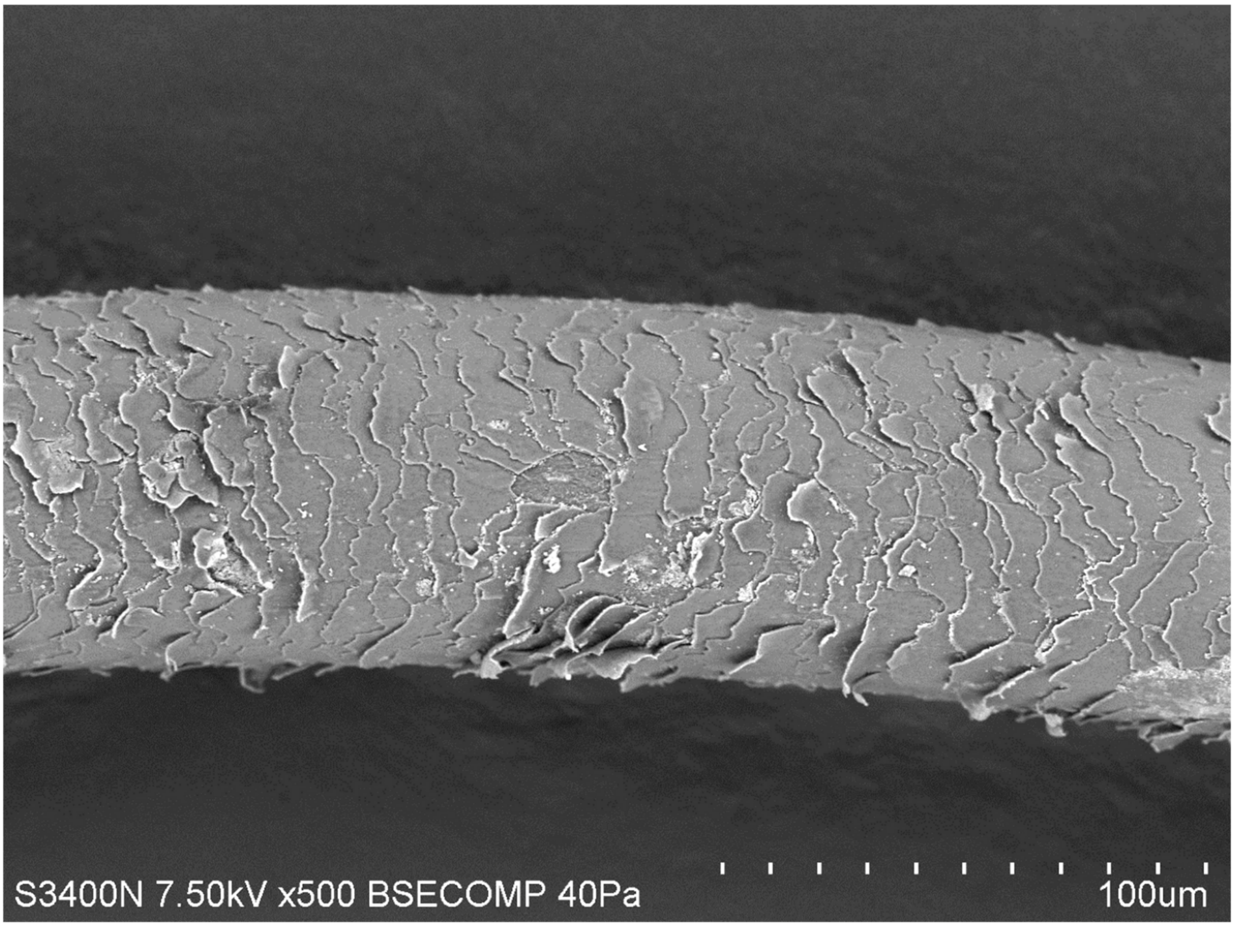

FIG.2

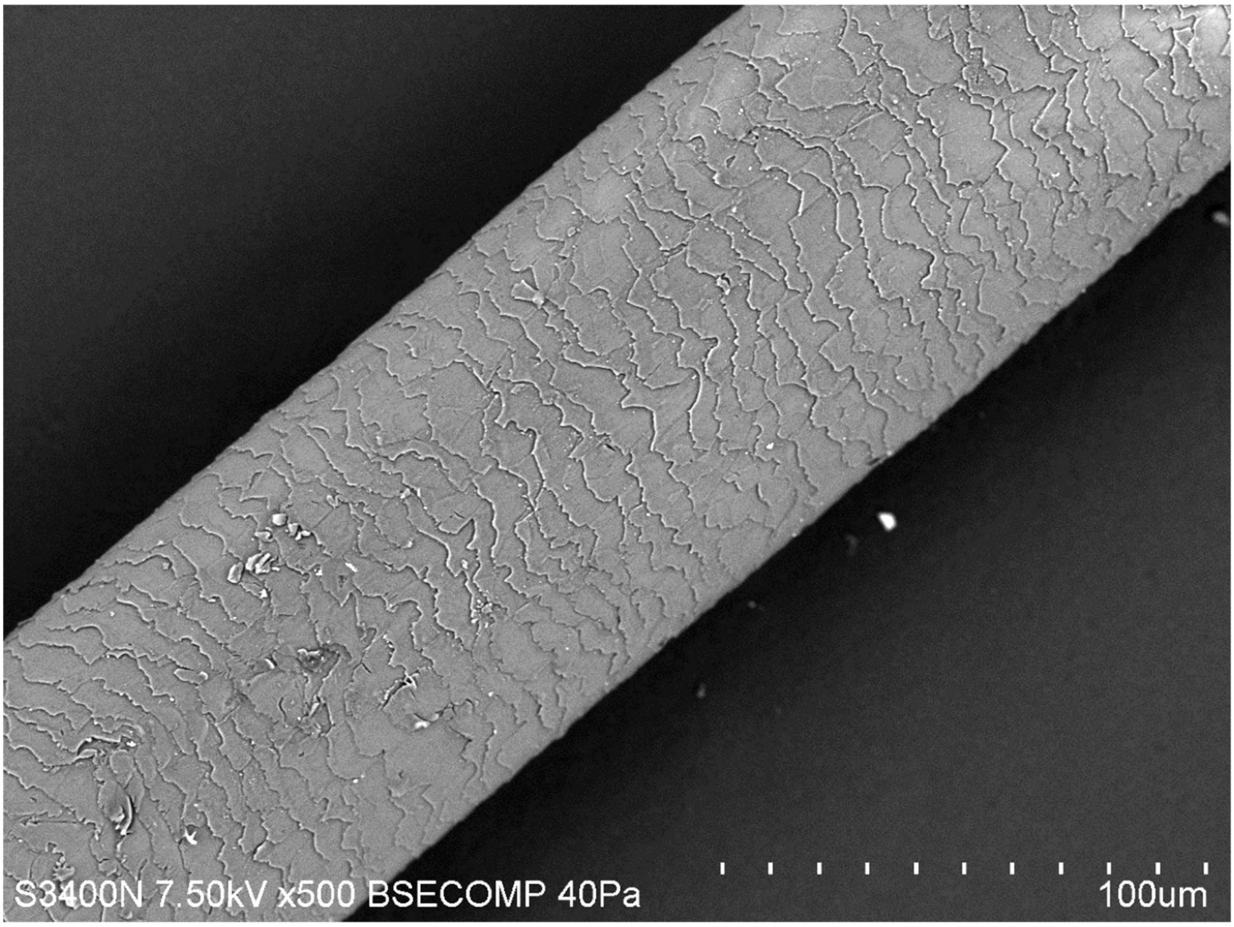

FIG.3

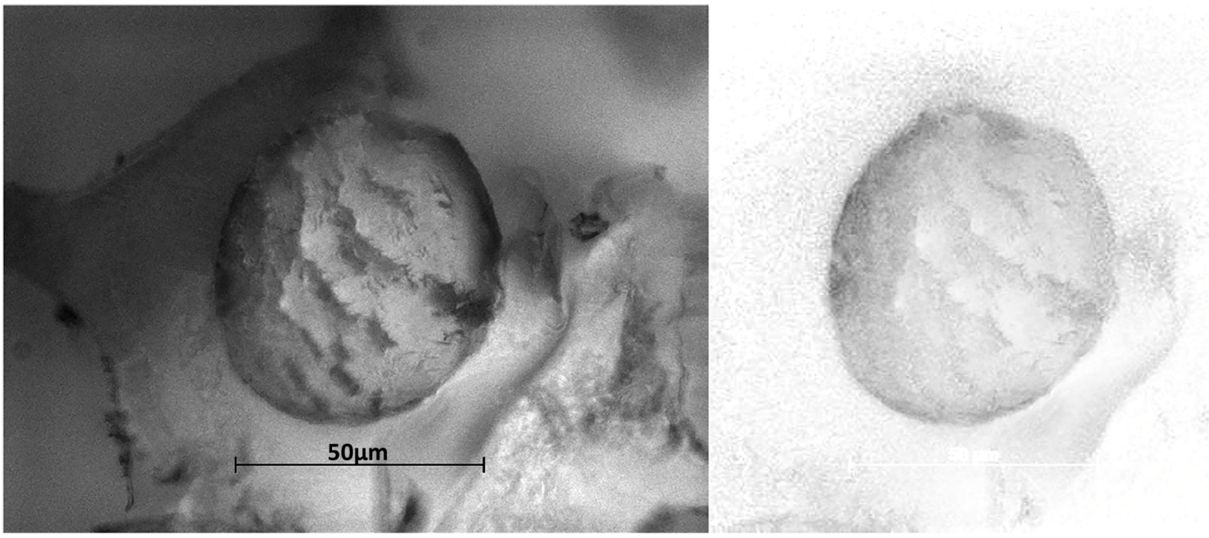


(A) (B)

FIG.4

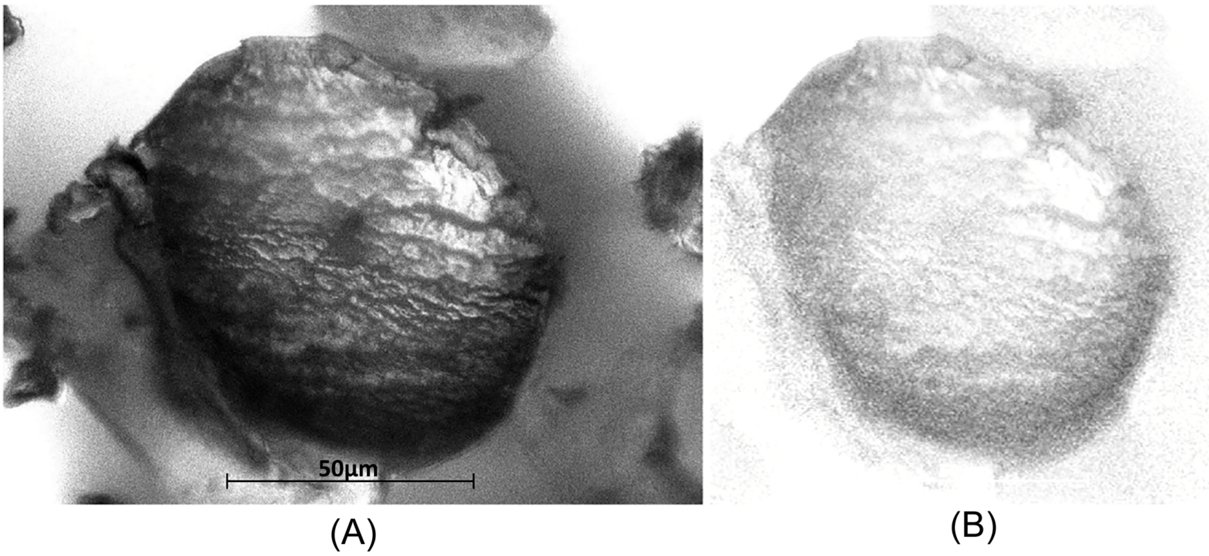


(A) (B)

FIG.5

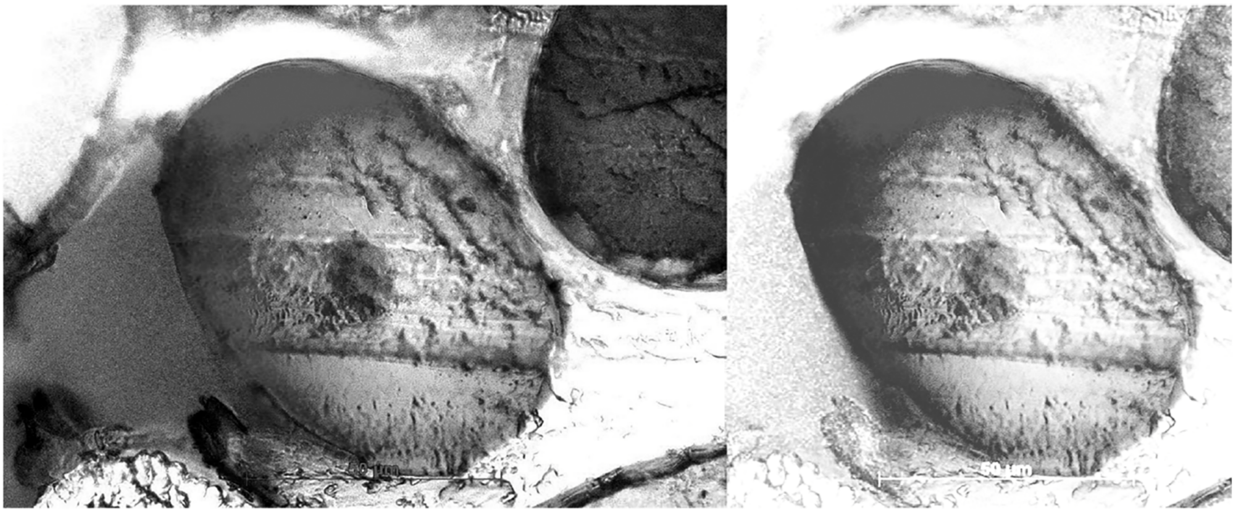

(A) (B)

FIG.6

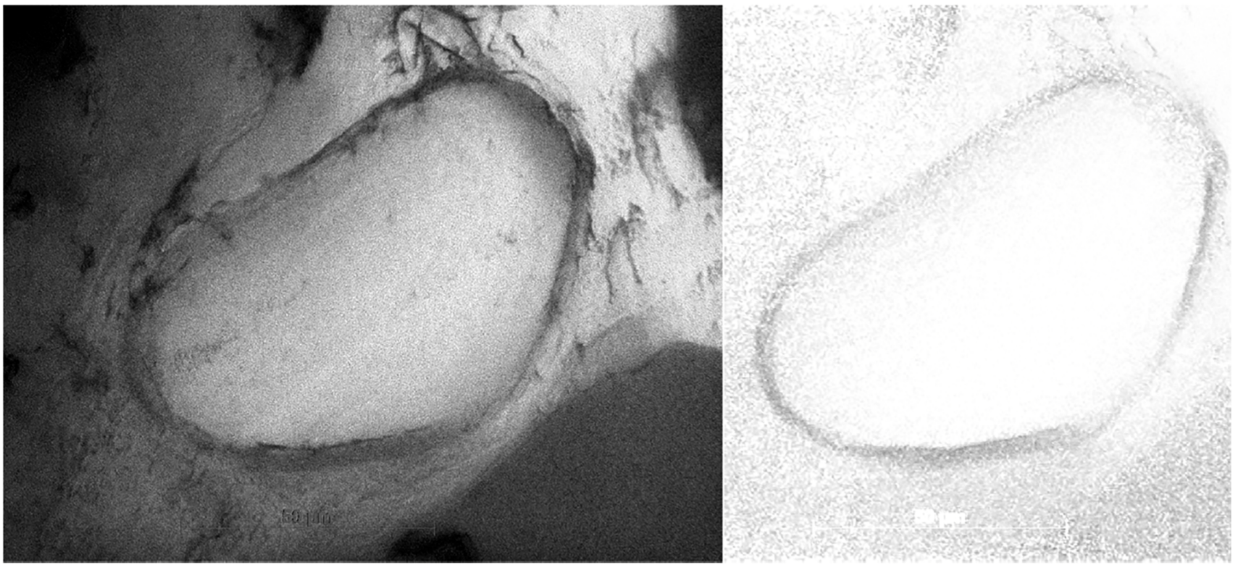

(A) (B)

FIG.7

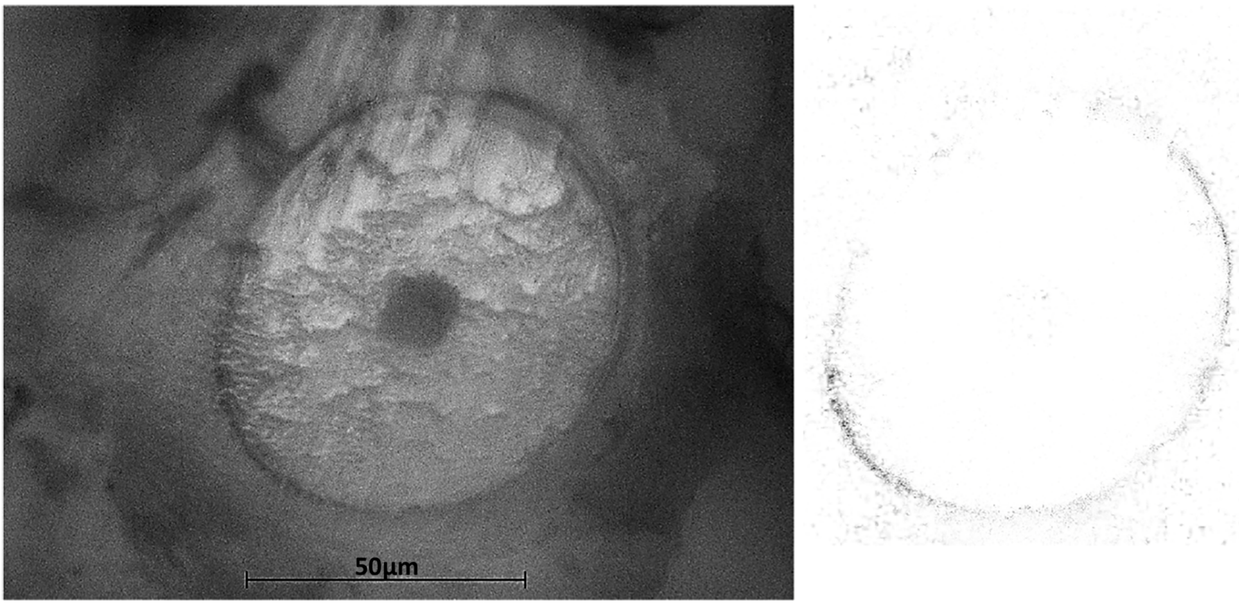


(A) (B)

## INTERNATIONAL SEARCH REPORT

International application No. **PCT/JP2024/017652**

### A. CLASSIFICATION OF SUBJECT MATTER

***A61K 8/41***(2006.01)i; ***A61K 8/36***(2006.01)i; ***A61K 8/44***(2006.01)i; ***A61Q 5/00***(2006.01)i
FI: A61K8/41; A61K8/44; A61K8/36; A61Q5/00

According to International Patent Classification (IPC) or to both national classification and IPC

### B. FIELDS SEARCHED

Minimum documentation searched (classification system followed by classification symbols)

A61K8/41; A61K8/36; A61K8/44; A61Q5/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br><br>Y | JP 2006-206488 A (NUUSU FIT KK) 10 August 2006 (2006-08-10)<br>claim 2, paragraphs [0001], [0005], [0008], [0010] | 1-13<br><br>14 |
| X | JP 2007-246467 A (YURIKA KK) 27 September 2007 (2007-09-27)<br>claim 3, paragraphs [0001], [0019], [0022], [0025] | 1-3, 5-13 |
| X | JP 2005-350481 A (HOYU CO., LTD.) 22 December 2005 (2005-12-22)<br>claims 1-2, paragraphs [0001], [0006], [0031] | 1-3, 5-10, 12 |
| Y | JP 2002-114653 A (HOYU CO., LTD.) 16 April 2002 (2002-04-16)<br>claims 1-2, paragraphs [0001], [0013] | 14 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 July 2024** | **30 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (July 2022)

**INTERNATIONAL SEARCH REPORT**
**Information on patent family members**

International application No.
**PCT/JP2024/017652**

| Patent document cited in search report | Publication date (day/month/year) | Patent family member(s) | Publication date (day/month/year) |
|---|---|---|---|
| JP 2006-206488 A | 10 August 2006 | (Family: none) | |
| JP 2007-246467 A | 27 September 2007 | KR 10-2007-0094467 A<br>CN 101036623 A<br>TW 200803906 A | |
| JP 2005-350481 A | 22 December 2005 | (Family: none) | |
| JP 2002-114653 A | 16 April 2002 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018070461 A
- JP 2003505405 A
- JP 2021534103 A
- WO 2020166678 A
- WO 2022225048 A